# EUROPEAN PATENT APPLICATION

(11) **EP 4 385 497 A1**
(43) Date of publication of application: **19.06.2024**
(21) Application number: 23153670.7
(22) Date of filing: 27.01.2023
(51) Int. Cl.: A61K 9/16, A61K 9/20, A61K 31/00

(54) **ANTIOXIDANT-FREE FIXED DOSE COMBINATION OF NETUPITANT AND PALONOSETRON**

(30) Priority: 12.12.2022 IN 202211071675
(71) Applicant: Alfred E. Tiefenbacher (GmbH & Co. KG), 22767 Hamburg (DE)
(72) Inventor: Staver, Ruslan, 22767 Hamburg (DE); Prathap, Vamshi Ramana, 502319 Telangana (IN); Chalamalasetti, Siva Ramanjaneyulu, 502319 Telangana (IN); Balasubramanian, Velusamy, 502319 Telangana (IN); Joshi, Abhay Ramakant, 502319 Telangana (IN)
(74) Representative: Hertin und Partner Rechts- und Patentanwälte PartG mbB

(57) **Abstract**

The invention relates to a pharmaceutical composition comprising a fixed dose combination of (a) Netupitant or salt or hydrate thereof, and (b) Palonosetron or salt or hydrate thereof, in a single pharmaceutical unit, wherein the pharmaceutical composition does not comprise an antioxidant. The invention further relates to a method of preparing a pharmaceutical composition in the form of a tablet, the method comprising: (i) wet granulating a dry mixture comprising Netupitant or a salt or hydrate thereof, filler, a binder, a disintegrant, and optionally a surfactant and/or a lubricant, with a solution comprising Palonosetron or a salt or hydrate thereof, and optionally a surfactant and/or a binder, to produce granules, (ii) blending the granules with a lubricant, a disintegrant and a glidant, and optionally a filler, and (iii) compressing of the lubricated granules to produce a tablet. The invention further relates to the pharmaceutical composition for use in the treatment and/or prevention of nausea and/or vomiting, preferably in the treatment and/or prevention of acute or delayed nausea and vomiting associated with chemotherapy, such as highly or moderately emetogenic cancer chemotherapy, for example cisplatin cancer chemotherapy.

## Description

The invention is in the field of pharmaceutical compositions comprising the active ingredients Netupitant or salt or hydrate thereof, and Palonosetron or salt or hydrate thereof.

The invention relates to a pharmaceutical composition comprising a fixed dose combination of (a) Netupitant or salt or hydrate thereof, and (b) Palonosetron or salt or hydrate thereof, in a single pharmaceutical unit, wherein the pharmaceutical composition does not comprise an antioxidant. The invention further relates to a method of preparing a pharmaceutical composition in the form of a tablet, the method comprising: (i) wet granulating a mixture comprising Netupitant or a salt or hydrate thereof, filler, a binder, a disintegrant, and optionally a surfactant and/or a lubricant, with a solution comprising Palonosetron or a salt or hydrate thereof, and optionally a surfactant and/or a binder, to produce granules, (ii) blending the granules with a lubricant, a disintegrant and a glidant, and optionally a filler, and (iii) compressing of the lubricated granules to produce a tablet.

The invention further relates to the pharmaceutical composition for use in the treatment and/or prevention of nausea and/or vomiting, preferably in the treatment and/or prevention of acute or delayed nausea and vomiting associated with chemotherapy, such as highly or moderately emetogenic cancer chemotherapy, for example cisplatin cancer chemotherapy.

### BACKGROUND OF THE INVENTION

Netupitant (INN) chemically known as 2-[3,5-bis(trifluoromethyl)phenyl]-N,2-dimethyl-N-[4-(2-methylphenyl)-6-(4-methyl-piperazin-1-yl)pyridin-3-yl]propenamide (CAS 290297-26-6), is an anti-nausea and anti-emetic agent from the group of substance P/neurokinin 1 (NK-1) receptor antagonists. Netupitant is discovered by Roche and out-licensed to Hesinn Healthcare in 2005.

Netupitant competitively binds to and blocks the activity of the neurokinin 1 (NK-1) receptor in the central nervous system (CNS). Substance P is a neurotransmitter of the neurokinin (tachykinin) family, which can be found in neurons innervating the brain-stem nucleus tractus solitarii and the area postrema and may be elevated in response to chemotherapy leading to nausea and vomiting. By binding to NK-1 receptors, Netupitant inhibits binding of substance P to the NK-1 receptors and thereby prevents acute and delayed-onset nausea and vomiting during chemotherapy due to cancer, *e.g.,* the delayed phase of nausea and vomiting that occurs after the first 24 hours after application of chemotherapy.

Palonosetron (INN) chemically known as (3aS)-2-[(3S)-1-azabicyclo[2.2.2]octan-3-yl]-3a,4,5,6-tetrahydro-3H-benzo[de]isoquinolin-1-one (CAS 135729-61-2), often used as Palonosetron hydrochloride, is an anti-nausea and anti-emetic agent from the group of serotonin-3 (5-HT3) receptor antagonists. Palonosetron was discovered by Synthex (now part of Roche group) and out-licensed in 1998 to Helsinn Healthcare.

Palonosetron acts antagonistically at peripheral and central 5HT3-receptors preventing binding of serotonin to the receptor, resulting in the prevention of serotonin-induced nausea and emesis, e.g., immediate nausea that occurs within the first 24 hours after the application of chemotherapeutic agents.

Netupitant and Palonosetron are approved as an oral combination product in the form of a hard capsule comprising 300 mg Netupitant and 0.5 mg Palonosetron by several authorities including the European Medicines Agency (EMA) and the U. S. Food and Drug Administration (FDA) and is marketed by Helsinn Healthcare SA or its local marketing partners as Akynzeo^{®}.

According to the European public assessment report (EPAR) Akynzeo^{®} is indicated in adults for the prevention of acute and delayed nausea and vomiting associated with highly emetogenic cisplatin-based cancer chemotherapy and the prevention of acute and delayed nausea and vomiting associated with moderately emetogenic cancer chemotherapy. The recommended dose for Akynzeo^{®} is one capsule taken orally one hour before starting chemotherapy.

EP 430 190 B1 (Syntex/Roche) first described the chemical structure of Palonosetron. EP 2 099 298 A1 (Helsinn Healthcare) describes amorphous and the polymorphous crystalline solid-state forms of Palonosetron including Form I and Form II of Palonosetron hydrochloride.

EP 1 035 115 B1 (Roche) first described the chemical structure of Netupitant and EP 1 776 342 B1 (Roche) discloses the synthesis of Netupitant. WO 16/46638 A2 (Helsinn Healthcare) describes three crystalline forms of Netupitant including Form I of Netupitant as the only disclosed true polymorphic form, and two pseudo-polymorphic forms: Form II of Netupitant (trifluoroethanol solvate) and Form III of Netupitant (formate salt).

The commercial formulation of Akynzeo^{®} is a hard capsule containing Netupitant and Palonosetron in separate pharmaceutical units, within a hard capsule. The hard capsule comprises three Netupitant tablets as individual pharmaceutical units, and Palonosetron in a soft capsule as a further separate pharmaceutical unit. The Netupitant tablets are each comprised of 100 mg Netupitant, microcrystalline cellulose, sucrose lauric acid esters (sucrose laurate), povidone K-30, croscarmellose sodium, colloidal hydrated silica, sodium stearyl fumarate and magnesium stearate. The Palonosetron soft capsule is comprised of (a) a capsule shell containing gelatin, glycerol, sorbitol, 1,4-sorbitan and titanium dioxide and (b) a capsule fill containing 0.5 mg of palonosetron (as hydrochloride, i.e., 0.56 mg palonosetron hydrochloride), glycerol monocaprylcaproate (type I), glycerol, polyglyceryl oleate, purified water and butylated hydroxyanisole (BHA) as antioxidant.

From the prior art it is evident that Netupitant as well as Palonosetron are susceptible to oxidative degradation during formulation within a final dosage form. Thus, previously described commercial formulations typically comprise the antioxidant butylated hydroxyanisole as one key excipient to prevent, in particular, oxidative degradation of Palonosetron.

Due to the chemical properties and reactivity of antioxidants, such substances can potentially be harmful to living cells and thus are associated with potential health risks when used in humans. The use of larger amounts of the antioxidant butylated hydroxyanisole, as used within the commercial Akyzeo^{®} product, has previously caused stomach and liver cancer in mice.

Furthermore, an induction of allergies and changes in immune responses have been observed in animal studies. Thus, butylated hydroxyanisole could be problematic in sensitive populations and widespread use may lead to hypersensitivity and allergies. Furthermore, a carcinogenic effect has been discussed previously.

Due to the above-mentioned reasons, according to the EMA and the scientific guideline "Excipients in the dossier for application for marketing authorization of a medicinal product", the inclusion of antioxidants such as butylated hydroxyanisole has to be justified and whenever possible be avoided, or at least used at the lowest possible level within the formulation maintaining stability of the formulation.

The formulation of the Palonosetron soft gel capsule within the commercial Akynzeo^{®} formulation is disclosed in EP 1 940 366 B1 (Helsinn Healthcare). EP 2 727 590 B1 discloses the dosage form of Akynzeo^{®} comprising an outer shell such as a hard capsule containing one or more Netupitant units such as tablets and one or more Palonosetron units such as a soft gel capsule. According to the EPAR, the selection of the dosage form of Akynzeo^{®} comprising separate pharmaceutical units of Netupitant and Palonosetron within one hard capsule was based on the large difference in dose size of the active ingredients (300 vs. 0.5 mg) and differences in physicochemical properties of Netupitant and Palonosetron, which made a co-formulation within a single pharmaceutical unit difficult. Within a solid formulation Palonosetron displayed reduced potency and Netupitant and Palonosetron had poor content uniformity. The large dose of 300 mg Netupitant further showed insufficient solubility in liquids suitable for filling into soft capsules. Thus, a soft capsule or a solid formulation comprising both Netupitant and Palonosetron could not be effectively formulated.

The formulation and production of dosage forms comprising different types of pharmaceutical units and formulations within one dosage form as within the commercial formulation of Akynzeo^{®} is typically a complex and time-consuming process, as the different types of pharmaceutical units need to be manufactured separately by distinct manufacturing processes and subsequently combined into one dosage form in an additional manufacturing step.

The formulation and manufacturing of soft gel capsules, as for Palonosetron within the commercial Akynzeo^{®} product, is typically time-consuming and/or requires many excipients and often specialized equipment. The manufacturing process of soft gel capsules usually consists of seven main steps, including fill mix preparation (dissolution of Palonosetron and excipients in the fill solution), gelatin mass preparation, encapsulation into soft capsules and lubrication, drying, size sorting, washing and bulk packaging. The manufacturing process of the Netupitant tablets within the commercial Akynzeo^{®} product consists of seven main steps including mixing of Netupitant with excipients, high shear wet granulation, drying, milling, blending with further excipients, compression and bulk packaging. The overall manufacturing process of Akynzeo^{®} hard capsules filled with Netupitant tablets and Palonosetron soft capsules is thus a relatively complicated time-consuming manufacturing process.

Furthermore, the commercial Akynzeo^{®} product is a large capsule with approx. 22 mm length and may lead to poor patient compliance due to difficulties in swallowing the large capsule.

Difficulties in swallowing tablets and capsules can be a problem for many individuals and can lead to a variety of adverse events and patient noncompliance with treatment regimens. It is estimated that over 16 million people in the United States have swallowing difficulties, also known as dysphagia. For these individuals, swallowing a tablet or a capsule can be particularly challenging. A survey of adults on difficulties in swallowing tablets and capsules suggests that this problem goes well beyond the patient population with clinically recognized dysphagia and may affect as many as 40 percent of Americans. Individuals who find it difficult to swallow tablets and capsules frequently cite the size as the main reason for the difficulty in swallowing.

Size and shape of tablets and capsules affect the transit of the product through the pharynx and esophagus and may directly affect a patient's ability to swallow a particular drug product. Larger tablets and capsules have been shown to have a prolonged esophageal transit time. This can lead to disintegration of the product in the esophagus and/or cause injury to the esophagus, resulting in pain and localized esophagitis and the potential for serious sequelae including ulceration, stricture, and perforation. Other adverse events such as pain, gagging, choking, and aspiration are related to swallowing difficulties in the oropharyngeal phase of swallowing and increasingly occur at larger tablet and capsule sizes.

A variety of other factors may affect a patient's ability to swallow a tablet or a capsule. For example, age could be a factor. Children and adolescents, as well as the elderly, are more likely to have difficulty swallowing tablets or capsules. Body position, fluid intake, and the presence of certain medical conditions may also affect a patient's ability to swallow tablets and capsules.

Patient compliance with medication regimens may be influenced by the size and shape of a tablet or capsule. Studies in humans have also suggested that oval or capsule-shaped tablets are easier to swallow than round tablets of the same weight. The largest dimension of a tablet or capsule should not exceed 22 mm. Generally, it is preferable that a tablet or capsule should not exceed 17 mm in its largest dimension.

The commercial Akynzeo^{®} product is a large capsule with 7.6 mm thickness and approx. 22 mm length, being the maximal allowed largest dimension for oral products. In view of the approved indication for the prevention of nausea and vomiting associated with cancer chemotherapy and the fact that the cancer patients are often taking multiple different drug products daily, the patients may in general have difficulties with swallowing drug products and thus reduced compliance, which may be further reduced by the large capsule size.

Despite the provision of a combined dosage form of Netupitant and Palonosetron, these formulations involve the use of antioxidants to provide chemical stability of the active ingredients and the final dosage form, which potentially bear health risks and should be avoided within pharmaceutical compositions whenever possible. Further, the formulations of the prior art involve complex, cost-intensive, and time-consuming manufacturing processes comprising multiple manufacturing steps. Further, these formulations for oral application are large in size, which causes difficulties in swallowing and thus affects patient compliance. Hence, further developments are required for improved and more efficient means for the formulation of combined pharmaceutical compositions of Netupitant and Palonosetron, which overcome the drawbacks of the prior art, preferably providing a stable formulation, avoiding oxidative degradation of the active ingredients Netupitant and Palonosetron, which is in line with the guidelines regarding the use of antioxidants within pharmaceutical compositions (e.g., of the EMA). Further developments are required for the formulation of combined pharmaceutical compositions of Netupitant and Palonosetron, employing a simplified and reliable composition and method to easily manufacture cost-effective and stable formulations with the desired properties for medical application.

### SUMMARY OF THE INVENTION

In light of the prior art the technical problem underlying the invention was the provision of improved or alternative means for pharmaceutical compositions comprising a fixed dose combination of Netupitant or salts or hydrates thereof and Palonosetron or salts of hydrates thereof, that are sufficiently stable and avoid the use of antioxidants.

A further object of the invention is to provide improved or alternative means for pharmaceutical compositions comprising a fixed dose combination of Netupitant or salts or hydrates thereof and Palonosetron or salts of hydrates thereof that show comparable or improved solubility and/or oral bioavailability in comparison to the available dosage forms, such as hard capsules containing Netupitant tablets and Palonosetron soft capsules.

A further object of the invention is to provide improved or alternative means for pharmaceutical compositions comprising a fixed dose combination of Netupitant or salts or hydrates thereof and Palonosetron or salts of hydrates thereof that are technically reliable, simple and/or cost-effective to manufacture.

A further object of the invention is to provide improved or alternative means for pharmaceutical compositions comprising a fixed dose combination of Netupitant or salts or hydrates thereof and Palonosetron or salts of hydrates thereof for oral application, that have a good swallowability and better patient compliance in comparison to the available dosage forms. In providing a solution to these problems, the invention seeks to avoid the disadvantages of the prior art.

These problems are solved by the features of the independent claims. Preferred embodiments of the present invention are provided by the dependent claims.

One aspect of the invention therefore relates to a pharmaceutical composition comprising a fixed dose combination of (a) Netupitant or salt or hydrate thereof, and (b) Palonosetron or salt or hydrate thereof, in a single pharmaceutical unit, wherein the pharmaceutical composition does not comprise an antioxidant.

The compositions of the present invention are free of antioxidants and are surprisingly found to have comparable or improved stability in comparison to the available dosage forms of the prior art, in particular with regards to the chemical stability of the active ingredients Netupitant and Palonosetron. As shown in the examples, surprisingly, it was further found that the stability of the compositions of the present invention is substantially improved in comparison to comparative pharmaceutical compositions, wherein an antioxidant was included.

The compositions of the prior art (e.g., the commercial formulation Akynzeo^{®}) comprise antioxidants in order to avoid oxidative degradation in particular of Palonosetron. However, due to the chemical reactive moieties of such antioxidants, there are concerns about associated health risks such as hypersensitivity, allergies, changes in immune response and cancerogenic risks. Thus, several health authorities including the EMA, recommend and/or require the avoidance or restriction to minimal concentrations of antioxidants in pharmaceutical products, such as oral dosage forms.

The inventive composition represents to the knowledge of the inventors the first stable combined pharmaceutical composition comprising Netupitant and Palonosetron that is free of antioxidants. The provision of the present composition thus represents an unexpected technical advance with respect to avoiding the usage of antioxidants within a combined formulations comprising these two APIs.

Surprisingly, the compositions of the present invention further comprise Netupitant and Palonosetron within a single pharmaceutical unit, which shows high content uniformity and stability. The inventive composition represents to the knowledge of the inventors the first effective pharmaceutical composition to employ Netupitant and Palonosetron in admixture, i.e., within a single pharmaceutical unit. The provision of the present composition thus represents an unexpected technical advance with respect to combination formulation of these two APIs.

To date, the prior art teaches that due to difficulties in handling associated with the large difference in dose size of the two active pharmaceutical ingredients (APIs), differences in physicochemical properties and content uniformity, effective formulations combinations of Netupitant and Palonosetron must be prepared by preparing separate pharmaceutical units for each API, which are subsequently combined into a hard capsule shell. This results in a time-consuming and cost-intensive manufacturing process comprising multiple steps for preparing the pharmaceutical units for each API and the subsequent combination.

The compositions of the present invention advantageously enable the formulation of Netupitant and Palonosetron within a single pharmaceutical unit and thus result in a simpler, less complicated, cost-effective and technical reliable manufacturing process over the prior art. In one embodiment of the invention, the pharmaceutical composition is in the form of a tablet.

Surprisingly, the compositions of the present invention comprising Netupitant and Palonosetron within a single pharmaceutical unit could - in contrast to the prior art - be prepared in the form of a solid dosage form such as a tablet. In preferred embodiments the tablet of the invention represents a simple and unexpectedly effective composition, considering the previous difficulties reported for the two APIs.

The prior art teaches, that due to the large differences in dose size and in particular differences in physicochemical properties it was to date not possible to formulate both APIs within a single solid dosage form such as a tablet, as Palonosetron displayed reduced potency and had poor content uniformity, and furthermore, no solvent was available suitable to dissolve both APIs, thus precluding the formulation of *e.g.,* soft capsules comprising both APIs within the capsule fill.

The compositions of the present invention in the form of a tablet comprising Netupitant and Palonosetron in a fixed dose combination have unexpectedly high content uniformity, high stability and comparable or improved solubility in comparison to the reference formulation Akynzeo^{®}. Further, the compositions of the present invention show a low variability with respect to dissolution and are thus advantageous with regards to lower variability subsequent benefits in absorption and bioavailability of the active ingredients. In contrast the reference formulation Akynzeo^{®}shows relatively high dissolution variability, which can be attributed to the formulation comprising the active ingredients in separate dosage forms and additional packaging within a hard capsule shell, influencing dissolution of the dosage form. Thus, the formulation of a tablet comprising both APIs in a fixed dose combination provides the advantage of a stable formulation with the desired properties for medical use, such as improved dissolution properties, and at the same time can be manufactured easily, technically reliably and cost-effectively. This combination of improved properties over the prior art would not have been expected by a skilled person in light of the teachings of the prior art.

In embodiments the pharmaceutical composition in the form of a tablet has a size of not more than 17 mm in its longest dimension, preferably not more than 15 mm, more preferably not more than 14 mm.

In embodiments the pharmaceutical composition in the form of a tablet has a size of equal to or less than 17 mm in its longest dimension, preferably equal to or less than 15 mm, more preferably equal to or less than 14 mm.

In embodiments the tablet has an oval or capsule shaped form.

The compositions of the present invention advantageously enable the formulation of Netupitant and Palonosetron within a tablet that is smaller in size than the dosage forms available in the prior art. Thus, the tablet of the present invention provides the advantage of easier swallowability when applied orally and thus higher patient compliance.

The commercial Akynzeo^{®} product is a large capsule with a length of approx. 22 mm. As the size and shape of oral dosage forms may directly affect swallowability and transit of the product through the pharynx and esophagus and in further view of the indication for the prevention of nausea and vomiting associated with cancer chemotherapy, patient compliance may be substantially reduced for Akynzeo^{®}. Within the formulations of the prior art Netupitant and Palonosetron need to be formulated in separate pharmaceutical dosage forms, which cannot be effectively packed within one dosage form, requiring e.g., a large hard capsule shell for packaging. In contrast within the formulations of the present invention, the active ingredients can be effectively formulated and compressed within one pharmaceutical unit in the form of a tablet, which is substantially smaller, have a lower weight and are easier to swallow than the formulations of the prior art. The provision of the present composition in the form of a tablet with smaller size thus represents an unexpected technical advantage of the prior art, resulting in easier and more reliable intake by patients and thus higher patient compliance.

In one embodiment of the invention, the (a) Netupitant or salt or hydrate thereof, and (b) Palonosetron or salt or hydrate thereof are present in the same granular phase.

In one embodiment of the invention, the Netupitant and the Palonosetron are present in combination in an intragranular phase.

The combination of the inventive APIs in admixture, in the same granular phase of a composition, is unexpected over the prior art. Surprisingly, the two APIs can be prepared in combination, for example using a dry blend comprising Netupitant, which is mixed and granulated with a liquid comprising Palonosetron, thus maintaining both APIs in a single (preferably intragranular) phase. In embodiments, lubricating the granules and subsequent pressing to a tablet enables a simple and effective composition. Achieving acceptable content uniformity and solubility of both APIs from such a composition could not have been expected from the prior art.

In one embodiment of the invention, the composition comprises an intragranular phase, comprising a filler, a binder and a disintegrant, and optionally a lubricant.

In one embodiment of the invention the filler is microcrystalline cellulose and/or mannitol, the binder is povidone, copovidone and/or corn starch, the disintegrant is croscarmellose sodium, sodium starch glycolate, crospovidone and/or pregelatinized starch, and the lubricant is magnesium stearate and/or sodium stearyl fumarate.

In one embodiment of the invention, the composition comprises an intragranular phase, comprising a surfactant.

In one embodiment of the invention, the surfactant is sodium lauryl sulfate (SLS), poloxamer, sucrose laurate, polysorbate 20, polysorbate 80, sorbitan monolaurate and/or sorbitan monooleate.

In one embodiment of the invention, the lubricant is magnesium stearate and/or sodium stearyl fumarate, the disintegrant is croscarmellose sodium, sodium starch glycolate, crospovidone and/or pregelatinized starch, the glidant is colloidal silicon dioxide, and the filler is microcrystalline cellulose and/or mannitol.

In one embodiment of the invention, the pharmaceutical composition does not comprise titanium dioxide (TiOz).

Advantageously the compositions of the present invention are free of titanium dioxide, as the commercial product Akynzeo^{®} uses titanium dioxide as opacifier and colorant in oral dosage forms, such as tablets, granulates, hard capsules and soft gel capsules. However, upon recent evaluation of the safety of titanium dioxide there are concerns regarding its genotoxicity. Thus, titanium dioxide is no longer considered safe as a food additive by the EMA and should be avoided or replaced by alternative excipients within pharmaceutical compositions. The present composition thus represents a technical advance over the prior art with respect to avoiding the usage of titanium dioxide within a combined formulation comprising Netupitant and Palonosetron.

In one embodiment of the present invention, the pharmaceutical composition does not comprise an animal or animal-derived product. In one embodiment of the present invention, the pharmaceutical composition does not comprise an excipient derived from animals. In one embodiment of the present invention, the pharmaceutical composition is vegan.

Advantageously the compositions of the present invention are free of excipients derived from animals. In contrast the commercial product Akynzeo^{®} comprises several animal-derived excipients including gelatin from bovine sources within the hard capsule shell and shellac within the printing ink. The composition of the present thus represents a technical advance over the prior art with respect to avoiding the usage of animal-derived excipients. The composition of the present invention is therefore also applicable for the growing proportion of the world's vegan population avoiding the consumption of animal-derived products. In addition, a vegan formulation also allows the use in patients who follow the Halal or Kosher diets and/or avoiding the consumption of certain animal-derived products due to the religious reasons. The formulation is thus in comparison to the formulations of the prior art advantageously applicable to a larger patient group.

In one embodiment of the invention, the pharmaceutical composition comprises intragranular and extragranular phases, wherein a disintegrant is present in both granular phases in mass ratio of 5:1 to 1:5 of disintegrant in the intragranular to disintegrant in the extragranular phase, preferably 2:1 to 1:2, more preferably 1:1, and is preferably croscarmellose sodium, sodium starch glycolate, crospovidone and/or pregelatinized starch. In one embodiment a disintegrant is present in both granular phases, preferably in a mass ratio of 5:1 to 1:5 of disintegrant in the intragranular to disintegrant in the extragranular phase, such as 5:1, 4:1, 3:1, 2:1, 1:1, 1:2, 1:3, 1:4 or 1:5, more preferably 2:1 to 1:2, more preferably 1:1.

Surprisingly, the compositions of the present invention show beneficial dissolution properties compared to the prior art formulation Akynzeo^{®}. The presence of a disintegrant in both granular phases in combination with the excipients disclosed above enables surprisingly beneficial dissolution, in particular of the typically less soluble API Netupitant. Thereby, the disintegrant present in the extragranular phase initially causes the tablet to break up into granules, whereas the granules further disintegrate due to the intragranular portion of disintegrant, resulting in advantageous release and dissolution of the active ingredients Netupitant and Palonosetron.

In one embodiment of the invention, the pharmaceutical composition comprises: (i) an intragranular combination, comprising Netupitant or a salt or hydrate thereof, present in an amount of 50-75 wt%, preferably 55-70 wt%, more preferably 58-68 wt%, Palonosetron or a salt or hydrate thereof, present in an amount of 0.05-0.5 wt%, preferably 0.08-0.3 wt%, more preferably 0.1-0.2 wt%, a filler, present in an amount of 2-40 wt%, preferably 5-35 wt%, more preferably 6-32 wt%, a binder present in an amount of 0.1-10 wt%, preferably 0.5-9 wt%, more preferably 0.8-8 wt%, a disintegrant present in an amount of 0.1-5 wt%, preferably 0.5-4 wt%, more preferably 0.8-3.8 wt%, optionally a surfactant in an amount of 0.1-5 wt%, preferably 0.5-4 wt%, more preferably 0.8-3.5 wt%, optionally a lubricant in an amount of 0.1-5 wt%, preferably 0.5-3 wt%, more preferably 0.8-1.5 wt%, and (ii) an extragranular component, comprising a lubricant, present in an amount of 0.1-5 wt%, preferably 0.5-3 wt%, more preferably 0.8-1.5 wt%, optionally a disintegrant, present in an amount of 0.1-5 wt%, preferably 0.5-4 wt%, more preferably 0.8-3.8 wt%, optionally a glidant, present in an amount of 0.1-5 wt%, preferably 0.5-3 wt%, more preferably 0.8-2.5 wt%, and optionally a filler, in an amount of 1-30 wt%, preferably 5-20 wt%, more preferably 10-15 wt%, wherein wt% values are based on the total weight of all components of the tablet.

All values provided above, for example the specific preferred values for each component, may vary by +/- 2 wt%, by +/- 1 wt%, or by +/- 0.5 wt%.

The below embodiments are also be considered to encompass further embodiments of the invention in which the indicated amounts of the components are employed. These embodiments are not limited by total weight of the compositions, but in some embodiments by the wt% values of each component and/or the presence of each component in the tablet, without limitation to absolute weight. The preferred weight range in wt% may be employed for any given feature as a defining feature of the composition, without inherent limitation to the specific weight employed in each example or potential combinations with other components in the formulation.

### Embodiments based on E1 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 88.09 | 19.58 | 2-40 |
| 3 | Povidone *Ph. Eur.* | Binder | 21.00 | 4.67 | 0.1-10 |
| 4 | Sodium Lauryl Sulfate *Ph. Eur.* | Surfactant | 15.00 | 3.33 | 0.1-5 |
| 5 | Croscarmellose sodium *Ph. Eur.* | Disintegrant | 4.50 | 1.00 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 4.50 | 1.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E2 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 88.09 | 19.58 | 2-40 |
| 3 | Povidone *Ph. Eur.* | Binder | 21.00 | 4.67 | 0.1-10 |
| 4 | Poloxamer 188 *Ph. Eur.* | Surfactant | 15.00 | 3.33 | 0.1-5 |
| 5 | Croscarmellose sodium *Ph. Eur.* | Disintegrant | 4.50 | 1.00 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 4.50 | 1.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E3 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 103.09 | 22.91 | 2-20 |
| 3 | Povidone *Ph. Eur.* | Binder | 21.00 | 4.67 | 0.1-10 |
| 4 | Croscarmellose sodium *Ph. Eur.* | Disintegrant | 4.50 | 1.00 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose **sodium** *Ph. Eur.* | Disintegrant | 4.50 | 1.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E4 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 73.09 | 16.24 | 2-20 |
| 3 | Povidone *Ph. Eur.* | Binder | 31.50 | 7.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 6.75 | 1.50 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 6.75 | 1.50 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E5 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 73.09 | 16.24 | 2-40 |
| 3 | Povidone *Ph. Eur.* | Binder | 22.50 | 5.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 11.25 | 2.50 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 11.25 | 2.50 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E6 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 73.09 | 16.24 | 2-20 |
| 3 | Povidone *Ph. Eur.* | Binder | 13.50 | 3.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 15.75 | 3.50 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 15.75 | 3.50 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E7 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 91.09 | 20.24 | 2-40 |
| 3 | Povidone *Ph. Eur.* | Binder | 13.50 | 3.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 6.75 | 1.50 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 6.75 | 1.50 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E8 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 55.09 | 12.24 | 2-40 |
| 3 | Povidone *Ph. Eur.* | Binder | 31.50 | 7.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 15.75 | 3.50 | 0.1-5 |

| **Stage-B (API solution):** | | | | | |
|---|---|---|---|---|---|
| 1 | Palonosetron Hydrochloride *In-house* | API | 0.57 | 0.13 | 0.05-5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 15.75 | 3.50 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E9 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 60.00 | 50-75 |
| 2 | Cellulose, microcrystalline *Ph. Eur.* | Filler | 64.72 | 12.94 | 2-40 |
| 3 | Mannitol *Ph. Eur.* | Filler | 64.72 | 12.94 | 2-40 |
| 4 | Povidone *Ph. Eur.* | Binder | 20.00 | 4.00 | 0.1-10 |
| 5 | Sodium Lauryl *Sulfate Ph. Eur.* | Surfactant | 15.00 | 3.00 | 0.1-5 |
| 6 | Croscarmellose sodium *Ph. Eur.* | Disintegrant | 10.00 | 2.00 | 0.1-5 |

| **Stage-B (API solution):** | | | | | |
|---|---|---|---|---|---|
| 1 | Palonosetron Hydrochloride *In-house* | API | 0.56 | 0.11 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 10.00 | 2.00 | 0.1-5 |
| 2 | Colloidal silicon dioxide *Ph. Eur.* | Glidant | 10.00 | 2.00 | 0.1-5 |
| 3 | Magnesium Stearate *Ph. Eur.* | Lubricant | 5.00 | 1.00 | 0.1-5 |
| **Tablet Weight (mg):** | | | 500.00 | 100.00 | |

### Embodiments based on E10 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| | | | | | |
| 1 | Netupitant *In-house* | API | 300.00 | 60.00 | 50-75 |
| 2 | Cellulose, microcrystalline *Ph. Eur.* | Filler | 32.36 | 6.74 | 2-40 |
| 3 | Mannitol *Ph. Eur.* | Filler | 97.08 | 19.42 | 2-40 |
| 4 | Povidone *Ph. Eur.* | Binder | 20.00 | 4.00 | 0.1-10 |
| 5 | Sodium Lauryl *Sulfate Ph. Eur.* | Surfactant | 15.00 | 3.00 | 0.1-5 |
| 6 | Croscarmellose sodium *Ph. Eur.* | Disintegrant | 10.00 | 2.00 | 0.1-5 |

| **Stage-B (API solution):** | | | | | |
|---|---|---|---|---|---|
| 1 | Palonosetron Hydrochloride *In-house* | API | 0.56 | 0.11 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 10.00 | 2.00 | 0.1-5 |
| 2 | Colloidal silicon dioxide *Ph. Eur.* | Glidant | 10.00 | 2.00 | 0.1-5 |
| 3 | Magnesium Stearate *Ph. Eur.* | Lubricant | 5.00 | 1.00 | 0.1-5 |
| **Tablet Weight (mg):** | | | 500.00 | 100.00 | |

### Embodiments E11 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 60.00 | 50-75 |
| 2 | Cellulose, microcrystalline *Ph. Eur.* | Filler | 97.08 | 19.42 | 2-40 |
| 3 | Mannitol *Ph. Eur.* | Filler | 32.36 | 6.47 | 2-40 |
| 4 | Povidone *Ph. Eur.* | Binder | 20.00 | 4.00 | 0.1-10 |
| 5 | Sodium Lauryl *Sulfate Ph. Eur.* | Surfactant | 15.00 | 3.00 | 0.1-5 |
| 6 | Croscarmellose sodium *Ph. Eur.* | Disintegrant | 10.00 | 2.00 | 0.1-5 |

| **Stage-B (API solution):** | | | | | |
|---|---|---|---|---|---|
| 1 | Palonosetron Hydrochloride *In-house* | API | 0.56 | 0.11 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 10.00 | 2.00 | 0.1-5 |
| 2 | Colloidal silicon dioxide *Ph. Eur.* | Glidant | 10.00 | 2.00 | 0.1-5 |
| 3 | Magnesium Stearate *Ph. Eur.* | Lubricant | 5.00 | 1.00 | 0.1-5 |
| **Tablet Weight (mg):** | | | 500.00 | 100.00 | |

### Embodiments based on E12 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 60.00 | 50-75 |
| 2 | Cellulose, microcrystalline *Ph. Eur.* | Filler | 77.22 | 15.44 | 2-40 |
| 3 | Mannitol *Ph. Eur.* | Filler | 77.22 | 15.44 | 2-40 |
| 4 | Povidone *Ph. Eur.* | Binder | 5.00 | 1.00 | 0.1-10 |
| 5 | Sodium Lauryl *Sulfate Ph. Eur.* | Surfactant | 5.00 | 1.00 | 0.1-5 |
| 6 | Croscarmellose sodium *Ph. Eur.* | Disintegrant | 10.00 | 2.00 | 0.1-5 |

| **Stage-B (API solution):** | | | | | |
|---|---|---|---|---|---|
| 1 | Palonosetron Hydrochloride *In-house* | API | 0.56 | 0.11 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 10.00 | 2.00 | 0.1-5 |
| 2 | Colloidal silicon dioxide *Ph. Eur.* | Glidant | 10.00 | 2.00 | 0.1-5 |
| 3 | Magnesium Stearate *Ph. Eur.* | Lubricant | 5.00 | 1.00 | 0.1-5 |
| **Tablet Weight (mg):** | | | 500.00 | 100.00 | |

### Embodiments based on E13 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Croscarmellose sodium *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Polysorbate 20 *Ph. Eur.* | Surfactant | 15.00 | 3.33 | 0.1-5 |
| 3 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | |
| 3 | Magnesium Stearate *Ph. Eur.* | Lubricant | 4.50 | 1.00 | |
| **Tablet Weight (mg):** | | | 450.00 | 100.00 | |

### Embodiments based on E14 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Croscarmellose sodium *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Polysorbate 80 *Ph. Eur.* | Surfactant | 15.00 | 3.33 | 0.1-5 |
| 3 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E15 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| **1** | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Croscarmellose sodium *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Sorbitan monolaurate *Ph. Eur.* | Surfactant | 15.00 | 3.33 | 0.1-5 |
| 3 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E16 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Croscarmellose sodium *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Sorbitan monooleate *Ph. Eur.* | Surfactant | 15.00 | 3.33 | 0.1-5 |
| 3 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E17 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 18.00 | 4.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E18 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 18.00 | 4.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Colloidal silicon dioxide *Ph. Eur.* | Glidant | 9.00 | 2.00 | 0.1-5 |
| 2 | Magnesium Stearate *Ph. Eur.* | Lubricant | 4.50 | 1.00 | 0.1-5 |
| **Tablet Weight (mg):** | | | 450.00 | 100.00 | |

### Embodiments based on E19 of Netupitant/Palonosetron 300 mg/0.5 mg tablets:

| **S. No** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Sodium Starch Glycolate *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Sodium Starch Glycolate *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E20 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Crospovidone *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Crospovidone *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E21 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Pregelatinized starch *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| | | | | | |
| 1 | Pregelatinized starch *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E22 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Povidone *Ph. Eur.* | Binder | 9.00 | 2.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Povidone *Ph. Eur.* | Binder | 9.00 | 2.00 | 0.1-10 |
| 3 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E23 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Copovidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 9.00 | 2.00 | 0.1-5 |

| **Stage-B (API solution):** | | | | | |
|---|---|---|---|---|---|
| | | | | | |
| 1 | Palonosetron Hydrochloride *In-house* | API | 0.56 | 0.12 | 0.0-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E24 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 84.94 | 18.88 | 2-40 |
| 3 | Corn Starch *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 9.00 | 2.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E25 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Blending):** | | | | | |
| 1 | Palonosetron Hydrochloride *In-house* | API | 0.56 | 0.12 | 50-75 |
| 2 | Cellulose, microcrystalline *Ph. Eur.* | Filler | 80.44 | 17.88 | 2-40 |

| **Stage-B (Blending & Compaction):** | | | | | |
|---|---|---|---|---|---|
| 1 | Netupitant *In-house* | API | 300.00 | 67.67 | 0.05-0.5 |
| 2 | Povidone *Ph. Eur.* | Binder | 18.00 | 4.00 | 0.1-10 |
| 3 | Sodium Lauryl Sulfate *Ph. Eur.* | Surfactant | 15.00 | 3.33 | 0.1-5 |
| 4 | Croscarmellose sodium *Ph. Eur.* | Disintegrant | 9.00 | 2.00 | 0.1-5 |
| 5 | Magnesium Stearate *Ph. Eur.* | Lubricant | 4.50 | 1.00 | 0.1-5 |

| **Stage-C (Lubrication):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium Stearate *Ph. Eur.* | Lubricant | 4.50 | 1.00 | 0.1-5 |
| **Tablet Weight (mg):** | | | 450.00 | 100.00 | |

### Embodiments based on E26 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Blending):** | | | | | |
| 1 | Palonosetron Hydrochloride *In-house* | API | 0.56 | 0.12 | 0.05-0.5 |
| 2 | Cellulose, microcrystalline *Ph. Eur.* | Filler | 84.94 | 18.88 | 2-40 |

| **Stage-B (Blending & Comilling):** | | | | | |
|---|---|---|---|---|---|
| 1 | Netupitant *In-house* | API | 300.00 | 67.67 | 50-75 |
| 2 | Povidone *Ph. Eur.* | Binder | 18.00 | 4.00 | 0.1-10 |
| 3 | Sodium Lauryl Sulfate *Ph. Eur.* | Surfactant | 15.00 | 3.33 | 0.1-5 |
| 4 | Croscarmellose sodium *Ph. Eur.* | Disintegrant | 18.00 | 4.00 | 0.1-5 |
| 5 | Colloidal silicon dioxide *Ph. Eur.* | Glidant | 9.00 | 2.00 | 0.1-5 |

| **Stage-C (Blending):** | | | | | |
|---|---|---|---|---|---|
| 1 | Magnesium Stearate *Ph. Eur.* | Lubricant | 4.50 | 1.00 | 0.1-5 |
| **Tablet Weight (mg):** | | | 450.00 | 100.00 | |

### Embodiments based on E27 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 80.44 | 17.88 | 2-40 |
| 3 | Povidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 9.00 | 2.00 | 0.1-5 |
| 6 | Sodium Stearyl Fumarate *Ph. Eur.* | Lubricant | 4.50 | 1.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Sodium Stearyl Fumarate *Ph. Eur.* | Lubricant | 4.50 | 1.00 | 0.1-5 |
| **Tablet Weight (mg):** | | | 450.00 | 100.00 | |

### Embodiments based on E28 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Cellulose, microcrystalline *Ph. Eur.* | Filler | 80.44 | 17.88 | 2-40 |
| 3 | Povidone *Ph. Eur.* | Binder | 18.00 | 4.00 | 0.1-10 |
| 4 | Sodium Lauryl *Sulfate Ph. Eur.* | Surfactant | 15.00 | 3.33 | 0.1-5 |
| 5 | Croscarmellose sodium *Ph. Eur.* | Disintegrant | 9.00 | 2.00 | 0.1-5 |
| 6 | Sodium Stearyl Fumarate *Ph. Eur.* | Lubricant | 4.50 | 1.00 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | 0.1-5 |
| 2 | Colloidal silicon dioxide *Ph. Eur.* | Glidant | 9.00 | 2.00 | 0.1-5 |
| 3 | Magnesium stearate Ph. Eur | Lubricant | 4.50 | 1.00 | 0.1-5 |
| **Tablet Weight (mg):** | | | 450.00 | 100.00 | |

### Embodiments based on E29 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.00 | 66.67 | 50-75 |
| 2 | Mannitol *Ph. Eur.* | Filler | 30.00 | 6.67 | 2-40 |
| 3 | Povidone *Ph. Eur.* | Binder | 18.00 | 4.00 | 0.1-10 |
| 4 | Croscarmellose sodium *Ph. Eur.* | Disintegrant | 14.00 | 3.11 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Polysorbate 80 *Ph. Eur.* | Surfactant | 15.00 | 3.33 | 0.1-5 |
| 3 | Purified water | Solvent | *q. s*. | - | |

| **Stage-C (Lubrication and blending):** | | | | | |
|---|---|---|---|---|---|
| 1 | Cellulose, microcrystalline *Ph. Eur.* | Filler | 49.94 | 11.10 | 1-30 |
| 2 | Croscarmellose sodium *Ph. Eur.* | Disintegrant | 14.00 | 3.11 | 0.1-5 |
| 3 | Colloidal silicon dioxide *Ph. Eur.* | Glidant | 4.00 | 0.89 | 0.1-5 |
| 4 | Sodium Stearyl Fumarate *Ph. Eur.* | Lubricant | 4.50 | 1.00 | 0.1-5 |
| **Tablet Weight (mg):** | | | 450.00 | 100.00 | |

### Embodiments based on E30 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 302.85 | 67.30 | 50-75 |
| 2 | Cellulose, microcrystalline *Ph. Eur.* | Filler | 19.89 | 4.42 | 2-40 |
| 4 | Mannitol *Ph. Eur.* | Filler | 68.20 | 15.16 | 2-40 |
| 3 | Povidone *Ph. Eur.* | Binder | 13.50 | 3.00 | 0.1-10 |
| 4 | Sodium Lauryl *Sulfate Ph. Eur.* | Surfactant | 4.5 | 1.00 | 0.1-5 |
| 5 | Croscarmellose sodium *Ph. Eur.* | Disintegrant | 11.25 | 2.50 | 0.1-5 |
| 6 | Sodium Stearyl Fumarate *Ph. Eur.* | Lubricant | 4.50 | 1.00 | 0.1-5 |

| **Stage-B (API solution):** | | | | | |
|---|---|---|---|---|---|
| 1 | Palonosetron Hydrochloride *In-house* | API | 0.57 | 0.13 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 11.25 | 2.50 | 0.1-5 |
| 2 | Colloidal silicon dioxide *Ph. Eur.* | Glidant | 9.00 | 2.00 | 0.1-5 |
| 3 | Magnesium stearate *Ph. Eur.* | Lubricant | 4.50 | 1.00 | 0.1-5 |
| **Tablet Weight (mg):** | | | 450.00 | 100.00 | |

### Embodiments based on E31 of Netupitant/Palonosetron 300 mg/0.5 mg tablets:

| **S. No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.96 | 66.22 | 50-75 |
| 2 | Cellulose, microcrystalline *Ph. Eur.* | Filler | 44.51 | 9.79 | 2-40 |
| 4 | Mannitol *Ph. Eur.* | Filler | 45.47 | 10.00 | 2-40 |
| 3 | Povidone *Ph. Eur.* | Binder | 13.50 | 2.97 | 0.1-10 |
| 4 | Sodium Lauryl *Sulfate Ph. Eur.* | Surfactant | 4.5 | 0.99 | 0.1-5 |
| 5 | Croscarmellose sodium *Ph. Eur.* | Disintegrant | 11.25 | 2.48 | 0.1-5 |
| 6 | Sodium Stearyl Fumarate *Ph. Eur.* | Lubricant | 4.50 | 0.99 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 11.25 | 2.48 | 0.1-5 |
| 2 | Colloidal silicon dioxide *Ph. Eur.* | Glidant | 9.00 | 1.98 | 0.1-5 |
| 3 | Magnesium stearate *Ph. Eur.* | Lubricant | 4.50 | 1.98 | 0.1-5 |
| **Tablet Weight (mg):** | | | 454.50 | 100.00 | |

### Embodiments based on E32 of Netupitant/Palonosetron 300 mg/0.5 mg tablets:

| **S.No.** | **Ingredients** | **Function** | **mg/tablet** | **% by weight of tablet** | **Preferred range % by weight** |
|---|---|---|---|---|---|
| **Stage-A (Granulation):** | | | | | |
| 1 | Netupitant *In-house* | API | 300.96 | 66.22 | 50-75 |
| 2 | Cellulose, microcrystalline *Ph. Eur.* | Filler | 67.24 | 14.79 | 2-40 |
| 4 | Mannitol *Ph. Eur.* | Filler | 22.74 | 5.00 | 2-40 |
| 3 | Povidone *Ph. Eur.* | Binder | 13.50 | 2.97 | 0.1-10 |
| 4 | Sodium Lauryl *Sulfate Ph. Eur.* | Surfactant | 4.5 | 0.99 | 0.1-5 |
| 5 | Croscarmellose sodium *Ph. Eur.* | Disintegrant | 11.25 | 2.48 | 0.1-5 |
| 6 | Sodium Stearyl Fumarate *Ph. Eur.* | Lubricant | 4.50 | 0.99 | 0.1-5 |

| **Stage-B (API solution):** | | | | | |
|---|---|---|---|---|---|
| 1 | Palonosetron Hydrochloride *In-house* | API | 0.56 | 0.12 | 0.05-0.5 |
| 2 | Purified water | Solvent | *q. s*. | - | |

| **Stage-C (Lubrication and blending):** | | | | | |
|---|---|---|---|---|---|
| | | | | | |
| 1 | Croscarmellose sodium *Ph. Eur.* | Disintegrant | 11.25 | 2.48 | 0.1-5 |
| 2 | Colloidal silicon dioxide *Ph. Eur.* | Glidant | 9.00 | 1.98 | 0.1-5 |
| 3 | Magnesium stearate *Ph. Eur.* | Lubricant | 4.50 | 1.98 | 0.1-5 |
| **Tablet Weight (mg):** | | | 454.50 | 100.00 | |

In one embodiment of the invention, the tablet is not coated.

In one embodiment of the invention, the tablet shows at least 75% dissolution of Netupitant in the dissolution test using the USP Apparatus II in 900 ml of 0.07 M phosphate buffer pH 6.8 containing 1% SDS with the paddle speed of 100 rpm after 75 minutes and/or at least 75% dissolution of Palonosetron using the USP Apparatus II in 500 ml of 0.01 N HCl with the paddle speed of 75 rpm after 45 minutes.

In one embodiment of the invention, the tablet shows content uniformity (CU) between 85% and 115% (based on assay by HPLC analysis) and the acceptance value (AV) less than 15 for Palonosetron according to the content uniformity test of the European Pharmacopeia (Ph. Eur.) 2.9.40.

In a further aspect, the invention relates to a pharmaceutical composition as described herein for use in the treatment and/or prevention of nausea and/or vomiting, preferably in the treatment and/or prevention of acute or delayed nausea and vomiting associated with chemotherapy, such as highly or moderately emetogenic cancer chemotherapy, for example cisplatin cancer chemotherapy.

The invention therefore also relates to corresponding methods of treatment and/or prevention, for example a method of treating a subject with nausea and/or vomiting, the method comprising administering a therapeutically effective amount of a pharmaceutical composition as described herein to the subject.

In further embodiments the nausea and/or vomiting is selected from the list of acute or delayed nausea and vomiting associated with chemotherapy, such as highly or moderately emetogenic cancer chemotherapy, for example cisplatin cancer chemotherapy.

In a further aspect the invention relates to a method of preparing a pharmaceutical composition in the form of a tablet, the method comprising (i) wet granulating a dry mixture comprising Netupitant or a salt or hydrate thereof, filler, a binder, a disintegrant, and optionally a surfactant and/or a lubricant, with a solution comprising Palonosetron or a salt or hydrate thereof, and optionally a surfactant and/or a binder, to produce granules, (ii) blending the granules with a lubricant, a disintegrant and a glidant, and optionally a filler, and (iii) compressing of the lubricated granules to produce a tablet.

Advantageously the pharmaceutical compositions of the present invention comprising Netupitant and Palonosetron in a fixed dose combination can be manufactured in a simple, technically reliable and cost-effective manufacturing process.

The simple and cost-efficient process for manufacturing of tablets comprising both Netupitant and Palonosetron of the repent invention relates to a single manufacturing process comprising direct granulation of a mixture of Netupitant and excipients with a Palonosetron solution, producing granules, which are subsequently compressed into a tablet. In contrast the prior art formulation Akynzeo^{®} requires a complex and time-consuming process, as separate and different types of pharmaceutical units need to be manufactured for Netupitant and Palonosetron by distinct manufacturing processes (Netupitant tablets and Palonosetron soft capsules). These pharmaceutical units subsequently need to be combined into one dosage form in an additional manufacturing step. The present invention therefore enables simpler, less complicated and more cost-effective methods for manufacturing of tablets comprising both Netupitant and Palonosetron in a single pharmaceutical unit that are comparable and improved with respect to stability, content uniformity and dissolution properties over the prior art formulation

The features above with respect to the method of preparing a pharmaceutical composition have a structural and functional outcome on the tablets and dispersions of the present invention, such that the tablets and dispersions can in some embodiments be described by features of or derived from the method of preparing, and vice versa.

### DETAILED DESCRIPTION OF THE INVENTION

Netupitant (INN) 2-[3,5-bis(trifluoromethyl)phenyl]-N,2-dimethyl-N-[4-(2-methylphenyl)-6-(4-methyl-piperazin-1-yl)pyridin-3-yl]propenamide (CAS 290297-26-6) and salts or hydrates thereof is an anti-nausea and anti-emetic agent from the group neurokinin 1 (NK-1) receptor antagonists. Netupitant is a white non-hygroscopic, crystalline solid, which is slightly soluble in water and freely soluble in organic solvents such as acetone, toluene and methanol.

The chemical structure of Netupitant is:

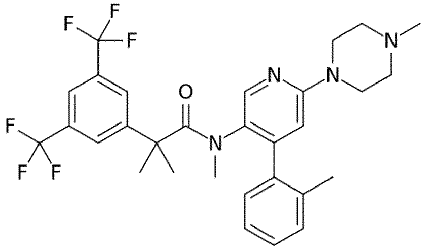

Netupitant and salts or hydrates thereof is a selective antagonist of the neurokinin 1 (NK-1) receptor. The activation of NK-1 receptors, which are broadly distributed in the central and peripheral nervous system (CNS and PNS) *e.g.,* in the gastrointestinal tract (GIT) and the area postrema and nucleus tractus soitarii, by substance P has been associated with nausea and emesis, in particular delayed nausea and emesis. By reducing and/or inhibiting binding of substance P to the receptors and thus inhibiting neuronal activation Netupitant prevents acute and delayed-onset nausea and emesis *e.g.,* during chemotherapy due to cancer.

Palonosetron (INN) (3aS)-2-[(3S)-1-azabicyclo[2.2.2]octan-3-yl]-3a,4,5,6-tetrahydro-3H-benzo[de]isoquinolin-1-one (CAS 135729-61-2) and salts or hydrates thereof such as Palonosetron hydrochloride is an anti-nausea and anti-emetic agent from the group of serotonin-3 (5-HT3) receptor antagonists. Palonosetron hydrochloride is a white, non-hydroscopic, crystalline powder, which is soluble in water, slightly soluble in polar organic solvents and insoluble in most apolar organic solvents.

The chemical structure of Palonosetron is:

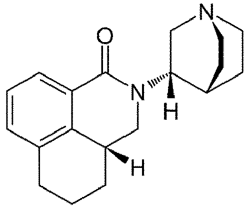

Palonosetron and salts or hydrates thereof is a 5-HT3 receptor antagonist. The release of serotonin also termed "5-HT" from enterochromaffin cells into the small intestine, e.g., upon application of chemotherapeutic agents is associated with nausea and emesis due to activation of 5-HT3 receptors on vagal afferents. In addition to activation of vagal afferents may lead to the release of serotonin in the area postrema, further promoting emesis. 5-HT3 receptor antagonists also termed "setrons" such as Palonosetron or salts or hydrated thereof inhibit this effect by antagonistically binding to peripheral and central 5-HT3 receptors, thereby reducing or hindering binding of serotonin to the 5-HT3 receptors.

Netupitant and Palonosetron hydrochloride are approved as a fixed dose combination containing 300 mg Netupitant and 0.5 mg Palonosetron by several authorities including the European Medicines Agency (EMA) and the U. S. Food and Drug Administration (FDA). It is marketed as as Akynzeo^{®} by Helsinn Healthcare or its local marketing partners.

Akynzeo^{®} is indicated for the prevention of acute and delayed nausea and vomiting associated with highly emetogenic cisplatin-based cancer chemotherapy and the prevention of acute and delayed nausea and vomiting associated with moderately emetogenic cancer chemotherapy.

Nausea and vomiting are frequent and usually uncharacteristic accompanying symptoms of diseases, for which a variety of causes can be considered including several types of viruses (*e.g*., Rota-, Adeno-, Corona- and Norovirus), bacteria (*e.g.*, Salmonella, Campylobacter, Shigella, Yersinia, Clostridium difficile and Vibrio cholerae), protozoa (e.g., amoebae, giardia), astric diseases, gallbladder affections, chronic pancreatitis, uremia, hepatic coma, vestibular vertigo, Meniere's disease, increased intracranial pressure (*e.g*., due to prior traumatic brain injury, intracerebral hemorrhage, meningitis and brain tumors), migraine attack, psychovegetative reaction to visual, olfactory and acoustic and auditory stimuli, kinetosis, hyperemesis gravidarium, depression, anxiety, anorexia, bulimia, excessive alcohol intake and emetogenic drugs or treatments (*e.g*., chemotherapy, digitalis glycosides, opioids, apomorphine and radiotherapy). Physiologically nausea and vomiting also termed "emesis" is associated with afferent impulses from the digestive tract to the vomiting center in the medulla oblongata, by excitation of chemoreceptors in the in the area postrema of the medulla oblongata, or by vestibular stimulation. Receptors involved in Nausea and emesis include D2-, 5-HT3, neurokinin-1 (NK-1), H1 receptors and m-cholinoceptors.

Upon administration of chemotherapy also termed "cancer chemotherapy" or "chemotherapeutic agents" "chemotherapeutics" or radiotherapy nausea and vomiting can occur either immediately within the first 24 h after administration (acute nausea and/or vomiting) or delayed after the first 24 h after administration (delayed nausea and/or vomiting). Chemotherapeutic agents can be classified according to their emetogenic potential. In the case of a combination of chemotherapeutic agents, the emetogenic potential of the therapy is evaluated according to the most emetogenic chemotherapeutic substance. Highly emetogenic chemotherapeutic agents induce emesis in >90% of patients and include without limitation cisplatin, cyclophosphamide ≥ 1500 mg/m², streptozotocin, carmustine, dactinomycin, mechlorethamine, streptozotocin and dacarbazine. Moderately emetogenic chemotherapeutic agents induce emesis in 30 to 90% of patients and include without limitation carboplatin, cyclophosphamide < 1500 mg/m², cytarabine, daunorubicin, doxorubicin, epirubicin, endoxane, ifosfamide, oxaliplatin, indarubicin, irinotecan, anthracyclines, azacytidine, oxaliplatin and bendamustine. Low emetogenic chemotherapeutic agents induce emesis in 10 to 30% of patients and include without limitation etoposide, gemcitabine, 5-fluorouracil (5-FU), docetaxel, paclitaxel, mitomycin, taxanes, cetuximab, bevacizumab, alemtuzumab, catumaxomab and panitumumab. Slightly emetogenic chemotherapeutic agents also termed minimal emetogenic chemotherapeutic agents induce emesis in < 10% of patients and include without limitation vinca-alkaloids and bleomycin.

The treatment and/or prevention of nausea and/or vomiting such as acute and/or delayed nausea and vomiting associated with chemotherapy, such as highly and/or moderately emetogenic cancer chemotherapy represent preferred embodiments of the medical use of the invention.

"Administration" or "treatment" as it applies to an animal, human, experimental subject, cell, tissue, organ, or biological fluid, refers to contact of a pharmaceutical, therapeutic, diagnostic agent, compound, or composition to the animal, human, subject, cell, tissue, organ, or biological fluid. "Administration" and "treatment" can refer, *e.g.,* to therapeutic, placebo, pharmacokinetic, diagnostic, research, and experimental methods. "Treatment," as it applies to a human, veterinary, or research subject, refers to therapeutic treatment, prophylactic or preventative measures, to research and diagnostic applications.

The invention encompasses administration of an effective amount of a chemical substance as described herein to a subject or patient in need thereof. "Effective amount" or "therapeutically effective amount" means an amount sufficient to elicit an appreciable biological response such as ameliorate a symptom or sign of a disorder or physiological condition when administered to a subject or patient. An effective amount for a particular patient or veterinary subject may vary depending on factors such as the condition being treated and the overall health and age of the patient. An effective amount can be the maximal dose or dosing protocol that avoids significant side effects or toxic effects. "Effective amount" also relates to an amount of the substance or pharmaceutical composition thereof, sufficient to allow or facilitate appreciable biological response such as the amelioration and of a symptom or sign of a disorder, condition, or pathological state.

The term "active ingredient" or "API" herein refers to a pharmaceutically active molecule Netupitant and/or Palonosetron as well as a pharmaceutically acceptable and/or therapeutically active salt thereof (*e.g*., Palonosetron hydrochloride). The term further refers to pharmaceutically acceptable and therapeutically active hydrates, esters, amides, metabolites, enantiomers, polymorphs, analogs, etc. that induce a desired pharmacological or physiological effect or induce a desired pharmacological or physiological effect upon transformation to a pharmaceutically active molecule in the organism. Terms like "active agent", "active pharmaceutical ingredient", "drug substance", may be used synonymously for "active ingredient". The term "pharmaceutically active molecule" herein refers to a molecule that induces a desired pharmacological or physiological effect in an organism and/or subject. Terms like "active drug", "active molecule", "therapeutically active molecule", "therapeutically active drug", may be used synonymously for "pharmaceutically active molecule".

The present invention relates further to salts of Netupitant and/or Palonosetron. The term "salt" refers to salts prepared by conventional means that include basic salts of inorganic and organic acids, including but not limited to acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate also termed esilate, fumarate, glucoheptanoate, gluconate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, saccharate, succinate, tartrate, thiocyanate, tosylate and undecanoate. For therapeutic use, salts of the compounds are those wherein the counter-ion is pharmaceutically acceptable. However, salts of acids which are non-pharmaceutically acceptable may also find use, for example, in the preparation or purification of a pharmaceutically acceptable compound.

The term "excipient" refers to a pharmacologically inactive component such as a filler, lubricant, binder, disintegrant, glidant, flavoring agent, sweetener, colorant, film formers, gelling agent, acid regulator, preservative agent, absorption enhancers, stabilizing agent and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient.

The excipients are described herein in some embodiments according to "wt%", or "percentage by weight" or "% by weight. The %wt values recite herein preferably relate to the percentage of material by weight present in the pharmaceutical composition in the form of a tablet.

According to the invention, a filler may be used as a bulking agent in solid dosage form such as tablets and granulates. Fillers used for tablets include but are not limited to sugars and sugar alcohols such as lactose, saccharose, glucose, mannitol, sorbitol, xylitol, and the like, oligo- and polysaccharides such as corn starch, rice starch, potato starch, wheat starch, modified starch derivatives, cellulose, microcrystalline cellulose (MCC), and the like, inorganic fillers such as calcium phosphate, calcium hydrogen phosphate, calcium carbonate, and mixtures thereof. According to the invention, the preferred filler is microcrystalline cellulose (MCC) and/or mannitol.

Microcrystalline cellulose (MCC) is obtained from cellulose by heating with mineral acids and subsequent mechanical comminution of the cellulose aggregates. The acid treatment causes limited hydrolysis, reducing the degree of polymerization (DP) to about 200-300 and increasing crystallinity. Subsequent spray drying of the dispersion yields powders of agglomerated cellulose crystallites suitable for direct tablet compression. Microcrystalline cellulose contributes to tablet hardness and reduces friability of a tablet due to its high plasticity. Further, tablet compression can be achieved at low compression pressure, resulting in reduced density of the tablet and thus fast disintegration and dissolution.

Mannitol (CAS No. 69-65-8) is a hexavalent sugar alcohol structurally derived from mannose and is naturally occurring among others in plants, algae and lichens. In the pharmaceutical industry mannitol is used as a filler, sweetener and binder. Mannitol can easily be compressed and compacted and is thus often used as filler to obtain stable tablets of small size. In the context of the invention mannitol is defined as a filler.

A glidant improves the flow properties of powder mixtures by reducing interparticle friction, Thus, glidants also improve the dosing accuracy. In particular, highly dispersed silicon dioxide is used as a glidant.

Silicon dioxide is the oxide of silicon. It is a natural substance that occurs for example in crystalline in quartz, granite and sand, and is used in the manufacture of glass. In the pharmaceutical industry, various grades of silicon dioxide find wide application as excipients. Thereby in particular, highly dispersed silicon dioxide also termed "colloidal silicon dioxide" is used as a glidant. The production of colloidal or highly dispersed silicon dioxide is carried out by flame hydrolysis from SiCl₄ resulting in submicroscopic amorphous spheres of approx. 7-16 nm in diameter and an extremely large surface area of about 200 m² /g. Addition of low amounts of about 0.5% are usually sufficient to achieve substantially improved flow properties of powders. According to the invention, the preferred glidant is colloidal silicon dioxide.

A disintegrant is an excipient used in tablets causing disintegration of the tablet, dissolution, and release of the active ingredient upon contact with moisture. Disintegrants can be classified into substances that increase capillarity, absorb moisture and swell, compounds which, when exposed to moisture flare up with gas evolution and substances that increase the wettability of the tablets (hydrophilizing agents).

Substances increasing capillarity include soy polysaccharides, alginic acid, crosslinked alginic acid, calcium alginate, natrium alginate, starches such as corn starch, pretreated starches such as pregelatinized starch, sodium carboxymethyl cellulose, crosslinked sodium carboxymethyl cellulose (also known as croscarmellose sodium), crosslinked calcium carboxymethyl cellulose (also known as carmellose calcium), crosslinked sodium carboxymethyl starch (also known as sodium starch glycolate), low-substituted hydroxypropylcellulose (L-HPC) and crosslinked polyvinylpyrrolidone (also known as crospovidone). Significant for the effect of this group is the exerted swelling pressure, the porosity and wettability of the tablet influencing the penetration of water into the tablet, which is a prerequisite for the disintegration process. These disintegrants are highly effective if they have high swellability, high swelling pressure and form a pore system in the tablet with sufficient wettability.

Highly effective disintegrants, which are more effective at much lower concentrations with greater disintegrating efficiency and mechanical strength compared with other disintegrants, are referred to as superdisintegrants. Superdisintegrants include crosslinked sodium carboxymethyl cellulose (also termed "croscarmelose sodium"), crosslinked calcium carboxymethyl cellulose (also termed "carmellose calcium") crosslinked sodium carboxymethyl starch (also termed "sodium starch glycolate"), and crosslinked polyvinylpyrrolidone (also termed "crospovidone"). These water-insoluble substances have a high swelling capacity as well as a high capillary activity and ensure spontaneous and complete disintegration without slime formation.

Compounds which, when exposed to moisture flare up with gas evolution include sodium hydrogen carbonate and hydrogen carbonate in combination with citric acid or tartaric acid. Tablets with such a disintegrant disintegrate rapidly due to carbon dioxide evolution upon an acid reaction. These disintegrants are commonly used for oral tablets or effervescent tablets.

Hydrophilizing agents include sodium lauryl sulfate, polysorbate, highly dispersed silicon dioxide and microcrystalline cellulose. The representatives of this group are technically not considered as disintegrants themselves. They rather enable disintegrants to become optimally effective. The tableting of lipophilic substances often causes considerable difficulties, since the wettability of such tablets is low, so that the incorporated disintegrants have no effect or only a very delayed effect. The addition of surface-active substances that hydrophilize the tablets /hydrophilizing agents) ensure penetration of water into the tablet and act on the incorporated disintegrants.

According to embodiments of the invention, the preferred disintegrant is a superdisintegrant, preferably croscarmellose sodium, sodium starch glycolate, crospovidone and/or pregelatinized starch.

Crospovidone (also known as cross-linked polyvinyl N-pyrrolidone, polyvinyl polypyrrolidone or PVPP) is an inert and insoluble white to light yellow free-flowing powder. It has hygroscopic or water-attracting properties with excellent swelling characteristics, making it useful as a disintegrant in pharmaceutical dosage forms. Crospovidone is not absorbed orally. Oral use of crospovidone is not usually associated with toxicity in normal use as a pharmaceutical excipient.

Sodium starch glycolate is the sodium salt of starch carboxymethyl ether. Starch glycolates are of rice, potato, wheat or corn origin. Sodium starch glycolate absorbs water rapidly, resulting in swelling which leads to rapid disintegration of tablets.

Croscarmelose sodium also termed "crosslinked sodium carboxymethyl cellulose" is the sodium salt of cross-linked carboxymethyl cellulose. It is a water-insoluble polysaccharide, which swells on contact with water commonly used as a disintegrant in solid dosage form such as tablets and granulates. Croscarmellose sodium is suitable for direct tableting as well as granulation.

Carmellose calcium, also termed "carboxymethylcellulose calcium" or "CMC calcium", is calcium salt of a polycarboxymethyl ether of cellulose. It is an effective tablet disintegrant and used similarly to croscarmellose sodium.

Pregelatinized starch (PGS) is generally considered as a filler (diluent), but may also have some disintegrant-like properties, therefore could be considered as "filler-disintegrant". PGS may also have binder-like properties. PGS is therefore a multi-functional excipient. PGS is a starch that has been chemically and/or mechanically processed to rupture all or part of the starch granules. This typically renders the starch flowable and directly compressible (Handbook of Pharmaceutical Excipients (Ed: Rowe)). Partially pregelatinized starch is commercially available. In some embodiments, pregelatinized starch contains 5% of free amylose, 15% of free amylopectin, and 80% unmodified starch. Pregelatinized starch is typically obtained from maize (corn), potato, or rice starch. Pregelatinized starch may be employed in granule or tablet formulations and shows multiple functions as a filler, disintegrant and/or binder. In the context of the present invention PGS is defined as disintegrant.

A lubricant is an excipient added to facilitate the tableting process, in particular with regard to compressing granules. Lubricants may facilitate flowability of a granule and/or powder to easily fill in a die, may reduce friction between granules and/or powder particles themselves and friction among a die, punch, granules and powder, and may facilitate tablet compressing and discharge from a die. Lubricants may thus prevent sticking of tablet compounds and tablets to tablet press punches and the die. Lubricants used for the formulation of tablets include magnesium stearate, calcium behenate, glycerol monostearate, stearic acid, sodium stearyl fumarate, talcum, hydrated vegetable fats such as hydrogenated castor oil, hydrogenated cottonseed oil and mixtures thereof. According to the invention, the preferred lubricant is magnesium stearate and/or sodium stearyl fumarate.

Magnesium stearate (CAS No. 557-04-0) is the magnesium salt of stearic acid and belongs to the lime soaps. Magnesium stearate is a salt consisting of a magnesium ion (Mg²⁺) and two stearates. Magnesium stearate is water insoluble. It has a lamellar crystal structure resulting in reduction of the internal friction and a very small particle size of 3 to 15 µm. Thus, magnesium stearate is a well suited as lubricant as it is attaching to the surface of other particles in a powder mixture, reducing interparticular friction and friction with external surfaces.

Sodium stearyl fumarate (CAS No. 4070-80-8) is synthetically produced by reacting stearyl alcohol with maleic anhydride. The product of this reaction then undergoes an isomerization step followed by salt formation to produce sodium stearyl fumarate. Sodium stearyl fumarate is used in oral pharmaceutical formulations as lubricant and is generally regarded as a nontoxic and nonirritant material.

According to embodiments of the invention a binder holds the ingredients in a tablet together and are preferably employed intragranularly during wet granulation. Binders are employed during granulation and for direct compression of tablets, in order to increase the cohesion of the powdery particles or granules during the compression ensuring that tablets and granules can be formed with required mechanical strength and defined hardness. Binders can further be used to act as processing aid during the granulation process. Binders are preferably macromolecular polar amorphous substances that exhibit isotropic deformation behavior due to their amorphous nature and provide optimum conditions for pouring into any available cavity during compression.

Binders can be classified into natural binders, semi-synthetic polymer binders and synthetic polymer binders. In the context of the present invention the expression "natural binder" refers to a natural polymer binder, or a salt thereof or an inorganic binder including starches, processed or pretreated starches or starch salts, *e.g.,* corn starch, potato starch, sodium starch, pregelatinized starch; alginic acid or salts thereof, *e.g.,* sodium alginate; gelatin; Guar gum; gum Arabic; Candelilla wax; Carnauba wax, dextran, sugars and hexitols such as lactose, mannitol, saccharose and inorganic calcium compounds such as calcium hydrogen phosphate and tribasic calcium phosphate.

In the context of the present invention the expression "semisynthetic polymeric binder" refers to a chemical derivative of natural polymer binder, including chemical derivatives of cellulose or starch, preferably selected from the group consisting of hydrolyzed starches such as dextran and maltodextrin, hydroxypropyl starch, hydroxypropyl cellulose (HPC, hyprollose), hydroxypropyl methyl cellulose (HPMC, Hypromellose), methyl cellulose (MC), and sodium carboxymethyl cellulose.

In the context of the present invention the expression "polymeric binder" refers to a fully chemically synthesized, non-natural polymer or co-polymer binder agent, preferably selected from the group of polyvinyl alcohol (PVA), polyethylene glycol (PEG), polyvinyl caprolactam-polyvinyl acetate, polyethylene glycol graft copolymer (marketed as Soluplus^{®}), polyethylene glycol-polyvinyl alcohol graft copolymer (PEG-PVA), povidone (PVP), and copovidone.

The binder may be present in the pharmaceutical composition in the form of a single constituent / ingredient or in the form of a mixture of constituents / ingredients. The preferred binder according to the present invention is povidone, copovidone and/or corn starch.

Povidone also termed "polyvinylpyrrolidone" (PVP) refers to linear polymers of N-vinyl-2-pyrrolidone also termed 1-vinyl-2-pyrrolidone. Povidone can include polymerization products with different molecular sizes or chain lengths. Overall, the molecular mass spectrum ranges from 10000-350000 Da. Povidone is usually present as a white to yellowish-white, highly hygroscopic, odorless powder or flake and is readily soluble in water and polar organic solvents such as alcohols, glycols and glycerol.

Copovidone is a copolymer of 1-vinyl-2-pyrrolidone and vinyl acetate in a ratio of 6:4. It has a lower hygroscopicity than pyrrolidone and can be used as a binder and as a film former for coating of tablets. In the context of the present invention copovidone is defined as a binder.

According to the present invention, surfactants (also termed as surface-active agents also wetting agents, emulsifying agents or suspending agents) are substances which adsorb onto the surfaces or interfaces of a system and lower the surface tension of the medium in which it is dissolved, and/or the interfacial tension with other phases. Surfactants are typically organic compounds that are amphiphilic, meaning they have a polar or hydrophilic (*i.e*., water-soluble) part and a non-polar (*i.e*., hydrophobic or lipophilic) part. The hydrophobic part of most surfactants is fairly similar, consisting of a hydrocarbon chain, which can be branched, linear, or aromatic. Surfactant molecules have either one tail or two; those with two tails are said to be double-chained. Most commonly, surfactants are classified according to their hydrophilic part. Hydrophilic groups can be anionic, cationic, zwitterionic or non-ionic in nature. Principles and applications of surfactants are known to the person skilled in the art and have been summarized in the literature, for example by the Book of Prof. Tharwat F. Tadros (Applied Surfactants: Principles and Applications; First published: 26 January 2005; Print ISBN:9783527306299; Online ISBN:9783527604814; DOI:10.1002/3527604812).

Suitable surfactants according to the present invention may include, but are not limited to, sodium lauryl sulfate (SLS), sucrose laurate, macrogoglycerol ricinoleate (commercially available as Kolliphor^{®} EL), polysorbate 20 polysorbate 40, polysorbate 60, polysorbate 80 (marketed as Tween^{®} 20, 40, 60, and 80, accordingly), polyoxyethylated oleic glycerides, polyoxyethylated glycol monoethers, polyoxyl 40 hydrogenated castor oil, polyethoxylated alkyl ethers (Brijs^{®} 35, 56, 78), lauroyl macrogol glyceride (Gelucire 44/14), Polyethoxylated fatty acid ester (Myrj^{®} 52, Solutol^{®} HS15), Polyethoxylated glycerides (Caprylo/caproil macrogolglyceride: Labrasol^{®}), polyoxyl castor oil derivatives (Polyoxyl 35 castor oil: Cremophor^{®} EL, polyoxyethylene polyoxypropylene block copolymers (poloxamers such as Poloxamer^{®} 188, Poloxamer^{®} 40, Kollisolv^{®} P 124 (Poloxamer 124 NF/EP)), saturated polyglycolized glycerides (Lauroyl macrogolglycerides: Gelucire^{®} 44/14 (Lauroyl Polyoxyl-32 glycerides), Stearoyl macrogolglycerides: Gelucire^{®} 50/13), Labrasol^{®} ALF (Caprylocaproyl macrogol-8 glycerides), linoleic macroglycerides, mixed glycerides of long chain fatty acids (Gelucire 33/01), oleic acid, glyceryl monostearate, Lecithin, Phosphatidylcholine and phosphatidylcholine mixtures (for example phosphatidylcholine mixtures in propylene glycol, medium chain triglycerides, ethanol), unsaturated polyglycolized glycerides (macrogolglycerides, such as Labrafil^{®} M1944 CS, Labrafil^{®} M2125CS), Sorbitan esters (such as sorbitan monooleate (Span^{®} 80) and sorbitan monolaurate (Span^{®} 20)), and polyethoxylated alkyl ethers (Brijs^{®} 30, 52, 72), and mixtures thereof. According to the invention, the preferred surfactant is sodium lauryl sulfate (SLS, also known as sodium dodecyl sulfate or SDS), poloxamer 188, sucrose laurate (also known as sucrose lauric acid esters), polysorbate 20, polysorbate 80, sorbitan monolaurate, sorbitan monooleate or mixtures thereof. The brand name/trademarks disclosed herein have become generally accepted synonyms of the respective chemical compounds and chemical names of the respective compounds can also be found by referring to technical information concerning the respective products.

The composition of the present invention may also comprise other excipients, such as pigments, and/or antioxidants, as may be desired.

An antioxidant is a chemical compound that slows down or completely prevents oxidation of other substances, e.g., of an active ingredient or an excipient within a pharmaceutical composition. According to the type of chemical mechanism of action, antioxidants can be classified into radical scavengers, reducing agents and antioxidant synergists. Radical scavengers are often compounds with sterically hindered phenolic groups that form inert stable radicals that do not react further, resulting in the termination of the oxidation reaction. Radical scavengers include without limitation tocopherols, tocopherol acetate, tocotrienols, polyphenolic compounds (e.g., flavonoids, anthocyanins, phytoestrogens, nordihydroguaiaretic acid), carotenoids (e.g., lycopene, beta carotene and lutein), butylated hydroxyanisole (BHA), butylated hydroxytoluene (BHT) and gallates (e.g., ethyl gallate, propyl gallate, octyl gallate and dodecyl gallate). Reducing agents have a low redox potential and are thus more likely to be oxidized than the substance to be protected. Reducing agents include without limitation ascorbic acid, sulfurous acid salts and organic sulfur-containing compounds (e.g., glutathione, cysteine and thiolactic acid). Antioxidant synergists enhance the effects of antioxidants for example, by regenerating depleted antioxidants. By complexing metal traces or creating an oxidation-inhibiting pH, synergists can enhance the antioxidant effect of a radical scavenger or reducing agent. Antioxidant synergists include for example sodium edetate.

The most commonly used pharmaceutical solid dosage forms today include granules, pellets, tablets and capsules. Tablets are solid pharmaceutical dosage forms containing drug substances with one or more excipients prepared by either compression or molding methods.

Methods for preparation of tablets include direct tableting (direct compression) and granulation including wet and dry granulation followed by compression. Tablet compression is carried out by a tablet press, which is a high-speed mechanical device. Two types of tablet presses are mainly used including eccentric presses and rotary presses. Both have two movable punches per functional unit (lower and upper punch), a die, and a hopper. These types of presses and their use are known to a person skilled in the art. The present invention preferably encompasses methods of wet granulation and subsequent production of a tablet.

In most cases, the active ingredients and excipients are granulated before tableting, *i.e.,* the powder particles are converted into granules. This results in a product with a larger particle size, which, compared to powder particles, has a better flowability. This ensures a continuous, uniform filling of the die of the tablet machine resulting in a constant tablet mass and high dosing accuracy. A granule is an asymmetric aggregate of powder particles that normally does not have a harmonious geometric shape and has an uneven, jagged or roughened surface resulting in good compressibility. Techniques for preparation of granulates include wet and dry granulation.

According to the present invention, wet granulation is the process of binding different powder particles together using an adhesive and/or liquid solution. Wet granulation techniques typically involve a granulation liquid, which is preferably a volatile solvent that is easy to remove by drying and should be non-toxic. The choice of liquid depends on the API and other excipients and can be elected as is required by a skilled person. Examples for wet granulation liquids relate to, for instance, water, ethanol, isopropanol or any other aqueous solution. The granulation liquid can further comprise an active pharmaceutical ingredient or an excipient. Various granulation technologies in batch and continuous modes may be employed, such as, without limitation, high shear granulators, fluid bed granulators, twin screw granulators, foam granulators and steam granulators. The process of wet granulation is known to a skilled person and can be adjusted depending on the characteristics of the powders and the available equipment. In the typical wet granulation method, the wet mass is forced through a sieve to produce wet granules that are subsequently dried. A subsequent screening stage breaks agglomerates of granules. Granules are then compressed into tablets.

The terms "intragranular" or "intragranular phase" refer to the components within granules or a granulate of the pharmaceutical composition, i.e., those used in preparing a mixture that is granulated, whereas the term "extragranular" or "extragranular phase" relates to additives or excipients added to the granulate, i.e., added to the intragranular phase after generation of initial granules.

As disclosed in the review article "Superdisintegrant: An overview" by Mohanachandran et al. (International Journal of Pharmaceutical Sciences Review and Research, Volume 6, Issue 1, January - February 2011; Article-022*),* a disintegrant used in granulated formulation processes can be more effective if used both "intragranularly" and "extragranularly" thereby acting to break the tablet up into granules and having the granules further disintegrate to release the drug substance into solution. There are three methods of incorporating disintegrating agents into the tablet: (a) internal addition (intragranular), (b) external addition (extragranular), (b) in both granular phases (partly internal and external). According to the present invention a disintegrant is present intragranularly, extragranularly or present in both granular phases, preferably in a mass ratio of 5:1 to 1:5, such as 5:1, 4:1, 3:1, 2:1, 1:1, 1:2, 1:3, 1:4 or 1:5, more preferably 2:1 to 1:2, more preferably 1:1.

According to the present invention the term "pharmaceutical unit" or "unit" refers to a pharmaceutical composition or mixture comprising one or more active ingredients and one or more excipients, wherein the one or more active ingredients and one or more excipients are physically present within one phase, *e.g.,* a powder, a granulate, an intragranular phase of a tablet, a tablet, a tablet prepared by compression of a granulate, a tablet prepared by direct compression of a powder, or a capsule. The pharmaceutical unit can for example be in the form of a powder, granulate, pellet, tablet or soft capsule. The pharmaceutical unit can, in embodiments, directly be administered to a subject as a dosage form, or further be processed to form a dosage form, for administration to a subject, e.g., compression of a granulate into a tablet. In embodiments, the pharmaceutical unit is a final dosage form suitable for administration to a subject. In embodiments, the pharmaceutical unit is a granulate, i.e., a single granular phase. According to the present invention the active ingredients Netupitant and Palonosetron or salts of hydrates thereof are present within a single pharmaceutical unit, preferably within a granulate, more preferably within an intragranular phase of a tablet prepared by compression of the granulate.

The terms "fixed dose" and "fixed dose combination" refer to a pharmaceutical composition comprising a defined amount of one or more active ingredients within one pharmaceutical unit or dosage form. In embodiments, the fixed dose combination is a combination of two or more final dosage forms suitable for direct administration to a subject.

### FIGURES

The invention is demonstrated by way of the figures disclosed herein. The figures provide support for a detailed description of potentially preferred, non-limiting embodiments of the invention.

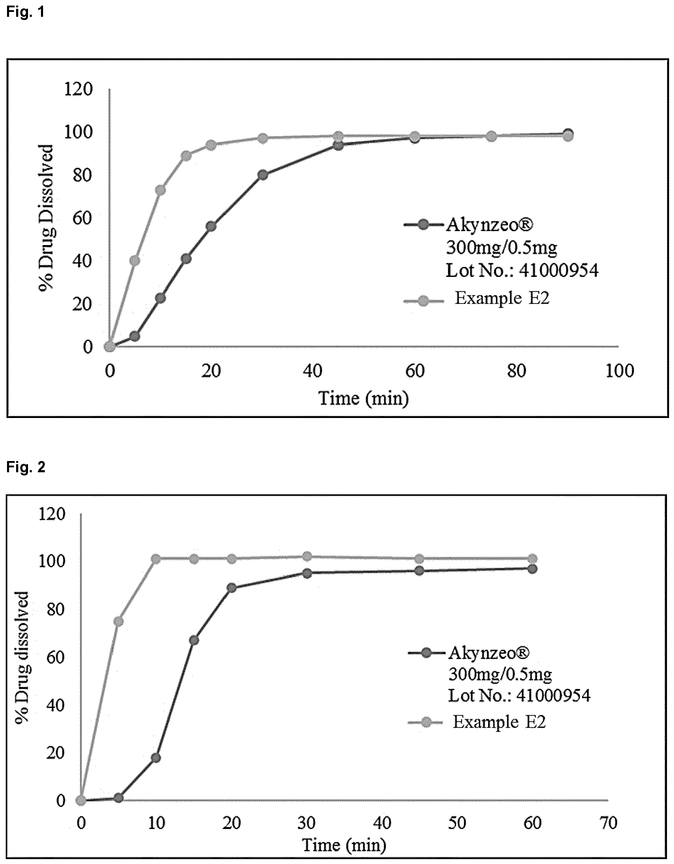

**Fig. 1****:** Comparative dissolution of Netupitant measured for example E2 and the reference product Akynzeo^{®}.
**Fig. 2****:** Comparative dissolution of Palonosetron hydrochloride measured for example E2 and the reference product Akynzeo^{®}.

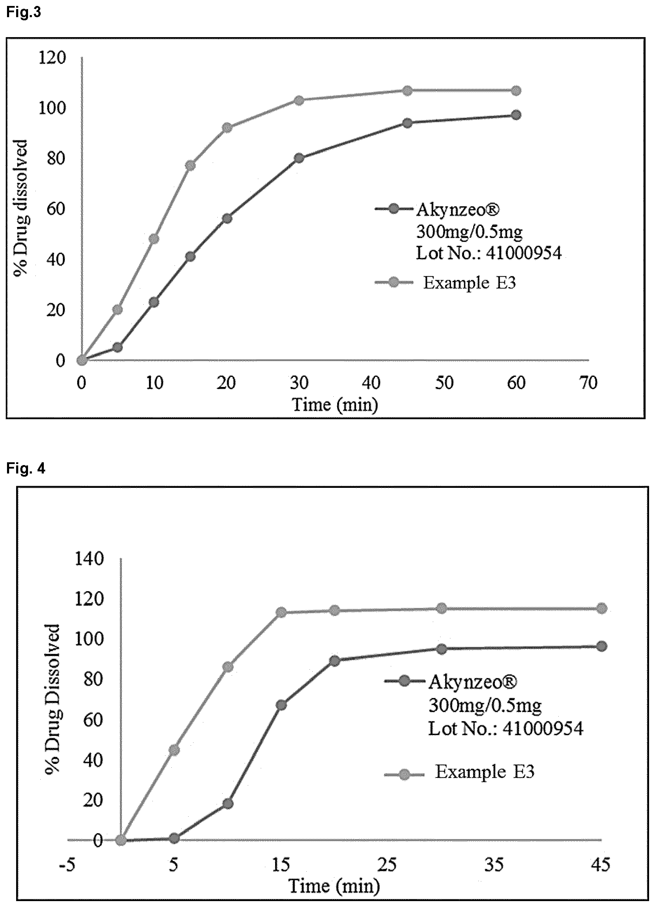

**Fig. 3****:** Comparative dissolution of Netupitant measured for example E3 and the reference product Akynzeo^{®}.
**Fig. 4****:** Comparative dissolution of Palonosetron hydrochloride measured for example E3 and the reference product Akynzeo^{®}.

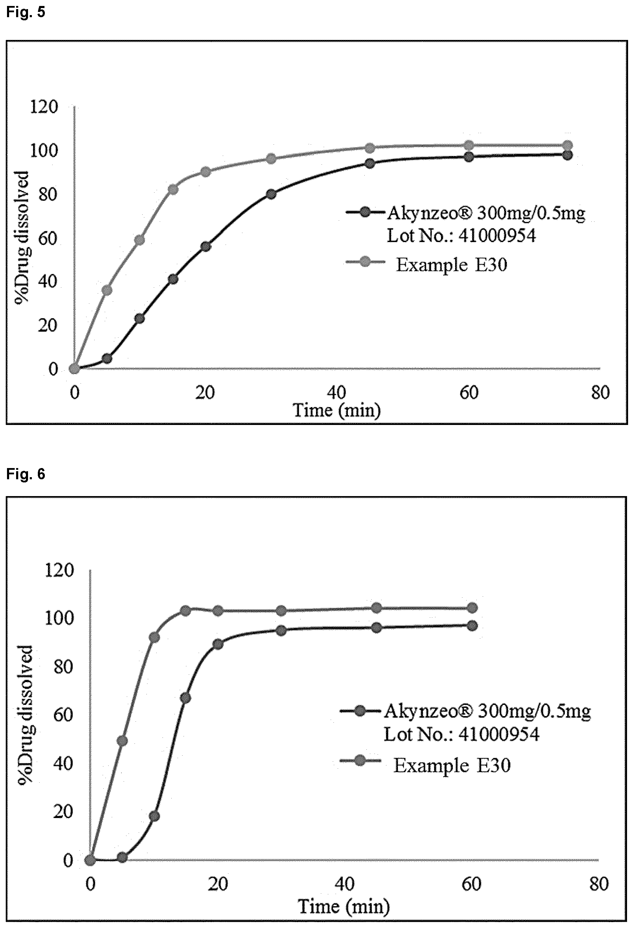

**Fig. 5****:** Comparative dissolution of Netupitant measured for example E30 and the reference product Akynzeo^{®}.
**Fig. 6****:** Comparative dissolution of Palonosetron hydrochloride measured for example E30 and the reference product Akynzeo^{®}.

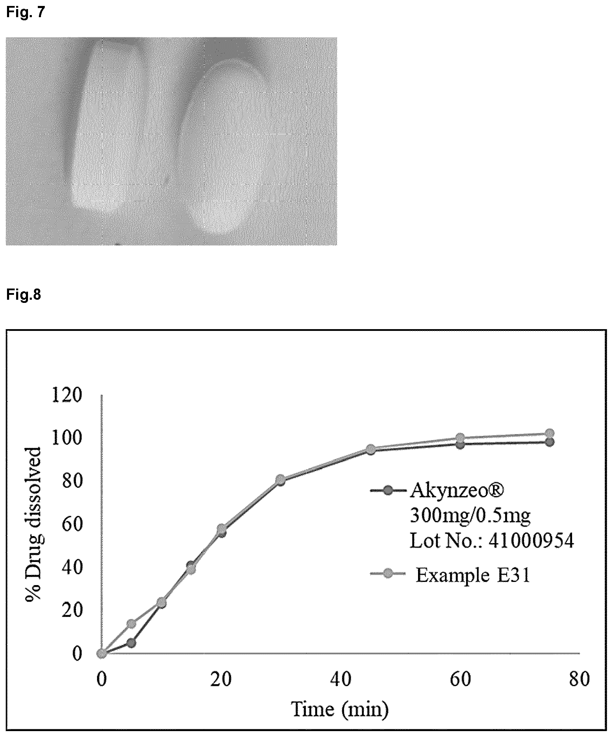

**Fig.7****:** Pictures of tablets prepared according to example E2. The tablets have a weight of 450 mg, a thickness of 6.09 to 6.16 mm, a width of 7.5 mm and a length of 14 mm.
**Fig. 8****:** Comparative dissolution of Netupitant measured for example E31 and the reference product Akynzeo^{®}.

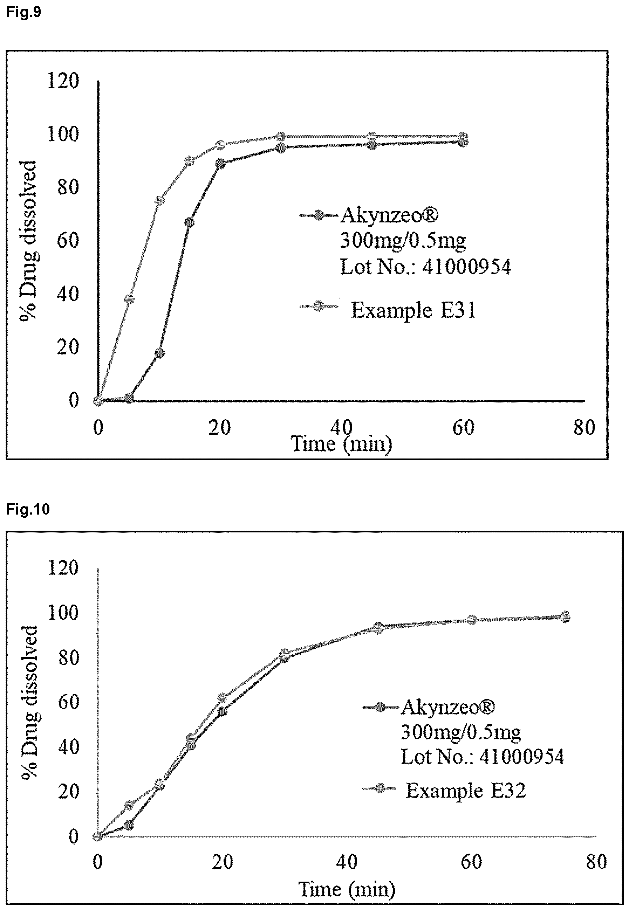

**Fig. 9****:** Comparative dissolution of Palonosetron hydrochloride measured for example E31 and the reference product Akynzeo^{®}.
**Fig. 10****:** Comparative dissolution of Netupitant measured for example E32 and the reference product Akynzeo^{®}.

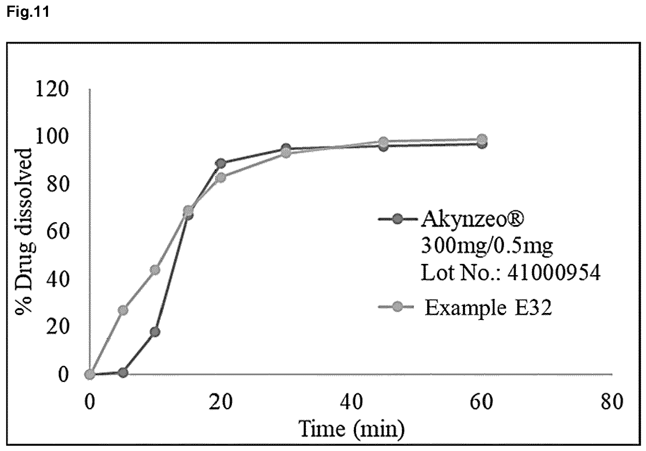

**Fig. 11****:** Comparative dissolution of Palonosetron hydrochloride measured for example E32 and the reference product Akynzeo^{®}.

### EXAMPLES

The invention is demonstrated by way of the examples disclosed herein. The examples provide technical support for a detailed description of potentially preferred, non-limiting embodiments of the invention.

### Part A - General manufacturing procedures

### General Manufacturing Procedure using wet granulation process (GP1):

**1. Dispensing:**
   1.1. Dispensed all the materials as per bill of materials.
**2. Sifting:**
   2.1. Co - sift the Stage-A material through ASTM # 20 mesh.
**3. API solution preparation:**
   3.1. Dissolve Stage-B material in purified water.
**4. Granulation:**
   4.1. Granulate the step 2.1 material with step 3.1 API solution using Rapid Mixer Granulator.
**5. Drying:**
   5.1. Dry the step 4.1 material using Rapid drier.
6. **Milling:**
   6.1. Mill the Step 5.1 material using quadro co mill fitted with 40G screen.
**7. Sifting of Extra** - **granular Material:**
   7.1. Sift the Stage-C material using ASTM #35 mesh.
**8. Blending:**
   8.1. Blend the step 6.1 material and step 7.1 material using suitable blender.
**9. Compression:**

   9.1. Compress the step 8.1 lubricated blend using suitable punches.

### General Manufacturing Procedure using dry granulation process (GP2):

**1. Dispensing:**
   1.1. Dispense all the material as per bill of materials.
**2. Sifting:**
   2.1. Co - Sift Palonosetron HCl IH and Cellulose, microcrystalline (Vivapur^{®} 102) through ASTM # 30 mesh.
**3. Blending:**

   3.1. Blend the step 2.1 material using suitable blender.
**4. Sifting:**
   4.1. Co - Sift step 3.1 and Stage - B material through ASTM # 20 mesh.
**5. Blending:**

   5.1. Blend the step 4.1 material using suitable blender.
**6. Dry Granulation:**
   6.1. Granulate the step 5.1 material using Chilsonator with appropriate parameters.
**7. Milling:**

   7.1. Mill the Step 6.1 material using OG mill or mill of Chilsonator fitted with 1.0 mm screen.
**8. Sifting of Extra** - **granular Material:**

   8.1. Sift the Stage - C material using ASTM #35 mesh.
**9. Blending:**

   9.1. Blend the step 6.1 material and step 5.1 material using suitable blender.
**10. Compression:**
   10.1. Compress the step 7.1 lubricated blend using suitable punches.

### General Manufacturing Procedure using direct compression process (GP3):

**1. Dispensing:**
   1.1. Dispense all the materials as per bill of materials.
**2. Sifting:**
   2.1. Co - sift Palonosetron HCl IH and Cellulose, microcrystalline (Vivapur^{®} 102) through ASTM # 30 mesh and resift the same through ASTM # 30 mesh
**3. Blending:**

   3.1. Blend the step 2.1 material using suitable blender.
**4. Milling:**
   4.1. Comill the step 3.1 material and stage-B material through appropriate screen of comil.
**5. Blending:**
   5.1. Blend the step 4.1 material using suitable blender.
**6. Sifting:**
   6.1. Sift the magnesium stearate using ASTM #35 mesh.
**7. Blending:**

   7.1. Blend the step 6.1 material and step 5.1 material using suitable blender.
**8. Compression:**
   8.1. Compress the step 7.1 lubricated blend using suitable punches.

### Part B - Comparative examples

**Comparative example C1 of Netupitant/Palonosetron 300 mg/0.5 mg tablets:** Comparative composition as tablet comprising Netupitant and Palonosetron as API and poloxamer 188 as surfactant, prepared by wet granulation. The composition further comprises propyl gallate as antioxidant.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 86.09 | 19.13 |
| 3 | Povidone K30 (Plasdone^{®} K-29/32) *Ph. Eur.* | Binder | 21.00 | 4.67 |
| 4 | Poloxamer 188 *Ph. Eur.* | Surfactant | 15.00 | 3.33 |
| 5 | Croscarmellose sodium *Ph. Eur.* | Disintegrant | 4.50 | 1.00 |
| 6 | Propyl gallate *Ph. Eur.* | Antioxidant | 2.00 | 0.44 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 4.50 | 1.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

### Part C - Inventive examples

Examples **E1 to E24** and **E27 to E32** are prepared according to the general procedure GP1. Example **E25** is prepared according to general procedure GP2. Example **E26** is prepared according to general procedure GP3.

**Example E1 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 88.09 | 19.58 |
| 3 | Povidone K30 (Plasdone^{®} K-29/32) *Ph. Eur.* | Binder | 21.00 | 4.67 |
| 4 | Sodium Lauryl Sulfate *Ph. Eur.* | Surfactant | 15.00 | 3.33 |
| 5 | Croscarmellose sodium *Ph. Eur.* | Disintegrant | 4.50 | 1.00 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 4.50 | 1.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E2 of NetupitantlPalonosetron 300 mg/0.5 mg tablets:** Inventive composition as tablet comprising Netupitant and Palonosetron as API and poloxamer 188 as surfactant, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 88.09 | 19.58 |
| 3 | Povidone K30 (Plasdone^{®} K-29/32) *Ph. Eur.* | Binder | 21.00 | 4.67 |
| 4 | Poloxamer 188 *Ph. Eur.* | Surfactant | 15.00 | 3.33 |
| 5 | Croscarmellose sodium *Ph. Eur.* | Disintegrant | 4.50 | 1.00 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 4.50 | 1.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E3 of NetupitantlPalonosetron 300 mg/0.5 mg tablets:** Inventive composition as tablet comprising Netupitant and Palonosetron as API, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of** tablet |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 103.09 | 22.91 |
| 3 | Povidone K30 (Plasdone^{®} K-29/32) *Ph. Eur.* | Binder | 21.00 | 4.67 |
| 4 | Croscarmellose sodium *Ph. Eur.* | Disintegrant | 4.50 | 1.00 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 4.50 | 1.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E4 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 73.09 | 16.24 |
| 3 | Povidone K30 (Plasdone^{®} K-29/32) *Ph. Eur.* | Binder | 31.50 | 7.00 |
| 4 | Sodium Lauryl Sulfate *Ph. Eur.* | Surfactant | 15.00 | 3.33 |
| 5 | Croscarmellose sodium *Ph. Eur.* | Disintegrant | 6.75 | 1.50 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 6.75 | 1.50 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E5 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 73.09 | 16.24 |
| 3 | Povidone K30 (Plasdone^{®} K-29/32) *Ph. Eur.* | Binder | 22.50 | 5.00 |
| 4 | Sodium Lauryl Sulfate *Ph. Eur.* | Surfactant | 15.00 | 3.33 |
| 5 | Croscarmellose sodium *Ph. Eur.* | Disintegrant | 11.25 | 2.50 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 11.25 | 2.50 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E6 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 73.09 | 16.24 |
| 3 | Povidone K30 (Plasdone^{®} K-29/32) *Ph. Eur.* | Binder | 13.50 | 3.00 |
| 4 | Sodium Lauryl Sulfate *Ph. Eur.* | Surfactant | 15.00 | 3.33 |
| 5 | Croscarmellose sodium *Ph. Eur.* | Disintegrant | 15.75 | 3.50 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 15.75 | 3.50 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E7 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 91.09 | 20.24 |
| 3 | Povidone K30 (Plasdone^{®} K-29/32) *Ph. Eur.* | Binder | 13.50 | 3.00 |
| 4 | Sodium Lauryl Sulfate *Ph. Eur.* | Surfactant | 15.00 | 3.33 |
| 5 | Croscarmellose sodium *Ph. Eur.* | Disintegrant | 6.75 | 1.50 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 6.75 | 1.50 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E8 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 55.09 | 12.24 |
| 3 | Povidone K30 (Plasdone^{®} K-29/32) *Ph. Eur.* | Binder | 31.50 | 7.00 |
| 4 | Sodium Lauryl Sulfate *Ph. Eur.* | Surfactant | 15.00 | 3.33 |
| 5 | Croscarmellose sodium *Ph. Eur.* | Disintegrant | 15.75 | 3.50 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 15.75 | 3.50 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E9 of NetupitantlPalonosetron 300 mg/0.5 mg tablets:** Inventive composition as tablet comprising Netupitant and Palonosetron as API and microcrystalline cellulose and mannitol as filler in a 50:50 weight ratio, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 60.00 |
| 2 | Cellulose, microcrystalline (Vivapur^{®} 101) *Ph. Eur.* | Filler | 64.72 | 12.94 |
| 3 | Mannitol (Pearlitol^{®} 25C) *Ph. Eur.* | Filler | 64.72 | 12.94 |
| 4 | Povidone K30 (Plasdone^{®} K-29/32) *Ph. Eur.* | Binder | 20.00 | 4.00 |
| 5 | Sodium Lauryl Sulfate *Ph. Eur.* | Surfactant | 15.00 | 3.00 |
| 6 | Croscarmellose sodium *Ph. Eur.* | Disintegrant | 10.00 | 2.00 |

| **Stage-B (API solution):** | | | | |
|---|---|---|---|---|
| 1 | Palonosetron Hydrochloride *In-house* | API | 0.56 | 0.11 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 10.00 | 2.00 |
| 2 | Colloidal silicon dioxide (Aerosil^{®} 200 Pharma) *Ph. Eur.* | Glidant | 10.00 | 2.00 |
| 3 | Magnesium Stearate *Ph. Eur.* | Lubricant | 5.00 | 1.00 |
| **Tablet Weight (mg):** | | | 500.00 | 100.00 |

**Example E10 of NetupitantlPalonosetron 300 mg/0.5 mg tablets:** Inventive composition as tablet comprising Netupitant and Palonosetron as API and microcrystalline cellulose and mannitol as filler in a 1:3 weight ratio, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 60.00 |
| 2 | Cellulose, microcrystalline (Vivapur^{®} 101) *Ph. Eur.* | Filler | 32.36 | 6.74 |
| 3 | Mannitol (Pearlitol^{®} 25C) *Ph. Eur.* | Filler | 97.08 | 19.42 |
| 4 | Povidone K30 (Plasdone^{®} K-29/32) *Ph. Eur.* | Binder | 20.00 | 4.00 |
| 5 | Sodium Lauryl Sulfate *Ph. Eur.* | Surfactant | 15.00 | 3.00 |
| 6 | Croscarmellose sodium *Ph. Eur.* | Disintegrant | 10.00 | 2.00 |

| **Stage-B (API solution):** | | | | |
|---|---|---|---|---|
| 1 | Palonosetron Hydrochloride *In-house* | API | 0.56 | 0.11 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 10.00 | 2.00 |
| 2 | Colloidal silicon dioxide (Aerosil^{®} 200 Pharma) *Ph. Eur.* | Glidant | 10.00 | 2.00 |
| 3 | Magnesium Stearate *Ph. Eur.* | Lubricant | 5.00 | 1.00 |
| **Tablet Weight (mg):** | | | 500.00 | 100.00 |

**Example E11 of NetupitantlPalonosetron 300 mg/0.5 mg tablets:** Inventive composition as tablet comprising Netupitant and Palonosetron as API and microcrystalline cellulose and mannitol as filler in a 3:1 weight ratio, prepared by wet granulation. The binder povidone is placed in the intragranular phase and the disintegrant croscarmellose sodium distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 60.00 |
| 2 | Cellulose, microcrystalline (Vivapur^{®} 101) *Ph. Eur.* | Filler | 97.08 | 19.42 |
| 3 | Mannitol (Pearlitol^{®} 25C) *Ph. Eur.* | Filler | 32.36 | 6.47 |
| 4 | Povidone K30 (Plasdone^{®} K-29/32) *Ph. Eur.* | Binder | 20.00 | 4.00 |
| 5 | Sodium Lauryl Sulfate *Ph. Eur.* | Surfactant | 15.00 | 3.00 |
| 6 | Croscarmellose sodium *Ph. Eur.* | Disintegrant | 10.00 | 2.00 |

| **Stage-B (API solution):** | | | | |
|---|---|---|---|---|
| 1 | Palonosetron Hydrochloride *In-house* | API | 0.56 | 0.11 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 10.00 | 2.00 |
| 2 | Colloidal silicon dioxide (Aerosil^{®} 200 Pharma) *Ph. Eur.* | Glidant | 10.00 | 2.00 |
| 3 | Magnesium Stearate *Ph. Eur.* | Lubricant | 5.00 | 1.00 |
| **Tablet Weight (mg):** | | | 500.00 | 100.00 |

**Example E12 of NetupitantlPalonosetron 300 mg/0.5 mg tablets:** Inventive composition as tablet comprising Netupitant and Palonosetron as API and microcrystalline cellulose and mannitol as filler in a 50:50 weight ratio, prepared by wet granulation. The binder povidone is placed in the intragranular phase and the disintegrant croscarmellose sodium distributed in the intragranular and extragranular phase at 50:50 weight ratio. Lower concentrations of the binder povidone and the surfactant sodium lauryl sulfate are used.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 60.00 |
| 2 | Cellulose, microcrystalline (Vivapur^{®} 101) *Ph. Eur.* | Filler | 77.22 | 15.44 |
| 3 | Mannitol (Pearlitol^{®} 25C) *Ph. Eur.* | Filler | 77.22 | 15.44 |
| 4 | Povidone K30 (Plasdone^{®} K-29/32) *Ph. Eur.* | Binder | 5.00 | 1.00 |
| 5 | Sodium Lauryl Sulfate *Ph. Eur.* | Surfactant | 5.00 | 1.00 |
| 6 | Croscarmellose sodium *Ph. Eur.* | Disintegrant | 10.00 | 2.00 |

| **Stage-B (API solution):** | | | | |
|---|---|---|---|---|
| 1 | Palonosetron Hydrochloride *In-house* | API | 0.56 | 0.11 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 10.00 | 2.00 |
| 2 | Colloidal silicon dioxide (Aerosil^{®} 200 Pharma) *Ph. Eur.* | Glidant | 10.00 | 2.00 |
| 3 | Magnesium Stearate *Ph. Eur.* | Lubricant | 5.00 | 1.00 |
| **Tablet Weight (mg):** | | | 500.00 | 100.00 |

**Example E13 of NetupitantlPalonosetron 300 mg/0.5 mg tablets:** Inventive composition as tablet comprising Netupitant and Palonosetron as API and polysorbate 20 (Tween^{®} 20) as surfactant, prepared by wet granulation. The binder povidone is placed in the intragranular phase and the disintegrant croscarmellose sodium distributed in the intragranular and extragranular phase at 50:50 weight ratio. The surfactant is placed in the binder solution.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Croscarmellose sodium *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Polysorbate 20 (Tween^{®} 20) *Ph. Eur.* | Surfactant | 15.00 | 3.33 |
| 3 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E14 of NetupitantlPalonosetron 300 mg/0.5 mg tablets:** Inventive composition as tablet comprising Netupitant and Palonosetron as API and polysorbate 80 (Tween^{®} 80) as surfactant, prepared by wet granulation. The binder povidone is placed in the intragranular phase and the disintegrant croscarmellose sodium distributed in the intragranular and extragranular phase at 50:50 weight ratio. The surfactant is placed in the binder solution.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Croscarmellose sodium *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Polysorbate 80 (Tween^{®} 80) *Ph. Eur.* | Surfactant | 15.00 | 3.33 |
| 3 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E15 of NetupitantlPalonosetron 300 mg/0.5 mg tablets:** Inventive composition as tablet comprising Netupitant and Palonosetron as API and sorbitan monolaurate (Span^{®} 20) as surfactant, prepared by wet granulation. The binder povidone is placed in the intragranular phase and the disintegrant croscarmellose sodium distributed in the intragranular and extragranular phase at 50:50 weight ratio. The surfactant is placed in the binder solution.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Croscarmellose sodium *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Sorbitan monolaurate (Span^{®} 20) *Ph. Eur.* | Surfactant | 15.00 | 3.33 |
| 3 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E16 of NetupitantlPalonosetron 300 mg/0.5 mg tablets:** Inventive composition as tablet comprising Netupitant and Palonosetron as API and sorbitan monooleate (Span^{®} 80) as surfactant, prepared by wet granulation. The binder povidone is placed in the intragranular phase and the disintegrant croscarmellose sodium distributed in the intragranular and extragranular phase at 50:50 weight ratio. The surfactant is placed in the binder solution.

| **S. No.** | **Ingredients** | **Function** | **mg/tablet** | **% by weight of tablet** | |
|---|---|---|---|---|---|
| **Stage-A (Granulation):** | | | | | |
| 1 | Netupitant *In-house* | API | 300.00 | 66.67 | |
| 2 | Cellulose, microcrystalline (Vivapur^{®} 101) *Ph. Eur.* | Filler | 84.94 | 18.88 | |
| 3 | Povidone K30 (Plasdone^{®} K-29/32) *Ph. Eur.* | Binder | 18.00 | 4.00 | |
| 4 | Croscarmellose sodium *Ph. Eur.* | Disintegrant | 9.00 | 2.00 | |

| **Stage-B (API solution):** | | | | | |
|---|---|---|---|---|---|
| 1 | Palonosetron Hydrochloride *In-house* | API | 0.56 | 0.12 | |
| 2 | Sorbitan monooleate (Span^{®} 80) *Ph. Eur.* | Surfactant | 15.00 | 3.33 | |
| 3 | Purified water | Solvent | *q. s*. | - | |

| **Stage-C (Lubrication and blending):** | | | | | |
|---|---|---|---|---|---|
| 1 | Croscarmellose sodium *Ph. Eur.* | Disintegrant | 9.00 | 2.00 | |
| 2 | Colloidal silicon dioxide (Aerosil^{®} 200 Pharma) *Ph. Eur.* | Glidant | 9.00 | 2.00 | |
| 3 | Magnesium Stearate *Ph. Eur.* | Lubricant | 4.50 | 1.00 | |
| **Tablet Weight (mg):** | | | 450.00 | 100.00 | |

**Example E17 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium in the extragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Sodium Lauryl Sulfate *Ph. Eur.* | Surfactant | 15.00 | 3.33 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 18.00 | 4.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E18 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is placed in the intragranular phase and the disintegrant croscarmellose sodium in the intragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Sodium Lauryl Sulfate *Ph. Eur.* | Surfactant | 15.00 | 3.33 |
| 5 | Croscarmellose sodium *Ph. Eur.* | Disintegrant | 18.00 | 4.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Colloidal silicon dioxide (Aerosil^{®} 200 Pharma) *Ph. Eur.* | Glidant | 9.00 | 2.00 |
| 2 | Magnesium Stearate *Ph. Eur.* | Lubricant | 4.50 | 1.00 |
| **Tablet Weight (mg):** | | | 450.00 | 100.00 |

**Example E19 of NetupitantlPalonosetron 300 mg/0.5 mg tablets:** Inventive composition as tablet comprising Netupitant and Palonosetron as API and sodium lauryl sulfate as surfactant and sodium starch glycolate as disintegrant, prepared by wet granulation. The binder povidone is placed in the intragranular phase and the disintegrant sodium starch glycolate distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Sodium Lauryl Sulfate *Ph. Eur.* | Surfactant | 15.00 | 3.33 |
| 5 | Sodium Starch Glycolate *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q.* s. | - |

| **Stage-C (Lubrication and blending):** | | | | |
|---|---|---|---|---|
| 1 | Sodium Starch Glycolate *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Core Tablet Weight (mg):** | | | 450.00 | 100.00 |

**Example E20 of NetupitantlPalonosetron 300 mg/0.5 mg tablets:** Inventive composition as tablet comprising Netupitant and Palonosetron as API and sodium lauryl sulfate as surfactant and crospovidone Type A (Polyplasdone^{®} XL) as disintegrant, prepared by wet granulation. The binder povidone is placed in the intragranular phase and the disintegrant crospovidone Type A (Polyplasdone^{®} XL) distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Sodium Lauryl Sulfate *Ph. Eur.* | Surfactant | 15.00 | 3.33 |
| 5 | Crospovidone Type A (Polyplasdone^{®} XL) *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Crospovidone Type A (Polyplasdone^{®} XL) *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E21 of NetupitantlPalonosetron 300 mg/0.5 mg tablets:** Inventive composition as tablet comprising Netupitant and Palonosetron as API and sodium lauryl sulfate as surfactant and pregelatinized starch as disintegrant, prepared by wet granulation. The binder povidone is placed in the intragranular phase and the disintegrant pregelatinized starch distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 18.00 | 4.00 |
| 4 | Sodium Lauryl Sulfate *Ph. Eur.* | Surfactant | 15.00 | 3.33 |
| 5 | Pregelatinized starch *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Pregelatinized starch *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E22 of NetupitantlPalonosetron 300 mg/0.5 mg tablets:** Inventive composition as tablet comprising Netupitant and Palonosetron as API and sodium lauryl sulfate as surfactant, prepared by wet granulation. The binder povidone is distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Povidone K30 (Plasdone^{®} K-29/32) *Ph. Eur.* | Binder | 9.00 | 2.00 |
| 4 | Sodium Lauryl Sulfate *Ph. Eur.* | Surfactant | 15.00 | 3.33 |
| 5 | Croscarmellose sodium *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Povidone K30 (Plasdone^{®} K-29/32) *Ph. Eur.* | Binder | 9.00 | 2.00 |
| 3 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E23 of NetupitantlPalonosetron 300 mg/0.5 mg tablets:** Inventive composition as tablet comprising Netupitant and Palonosetron as API and sodium lauryl sulfate as surfactant and copovidone as binder, prepared by wet granulation. The binder povidone is placed in the intragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Copovidone (Kollidon^{®} VA64) *Ph. Eur.* | Binder | 18.00 | 4.00 |
| 4 | Sodium Lauryl Sulfate *Ph. Eur.* | Surfactant | 15.00 | 3.33 |
| 5 | Croscarmellose sodium *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E24 of NetupitantlPalonosetron 300 mg/0.5 mg tablets:** Inventive composition as tablet comprising Netupitant and Palonosetron as API and sodium lauryl sulfate as surfactant and corn starch as binder, prepared by wet granulation. The binder povidone is placed in the intragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 84.94 | 18.88 |
| 3 | Corn Starch *Ph. Eur.* | Binder | 18.00 | 4.00 |
| 4 | Sodium Lauryl Sulfate *Ph. Eur.* | Surfactant | 15.00 | 3.33 |
| 5 | Croscarmellose sodium *Ph. Eur.* | Disintegrant | 9.00 | 2.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E25 of NetupitantlPalonosetron 300 mg/0.5 mg tablets:** Inventive composition as tablet comprising Netupitant and Palonosetron as API and sodium lauryl sulfate as surfactant and corn starch as binder, prepared by dry granulation.

| **S. No.** | **Ingredients** | **Function** | **mg/tablet** | **% by weight of tablet** |
|---|---|---|---|---|
| **Stage-A (Blending):** | | | | |
| 1 | Palonosetron Hydrochloride *In-house* | API | 0.56 | 0.12 |
| 2 | Cellulose, microcrystalline (Vivapur^{®} 102) *Ph. Eur.* | Filler | 80.44 | 17.88 |

| **Stage-B (Blending & Compaction):** | | | | |
|---|---|---|---|---|
| 1 | Netupitant *In-house* | API | 300.00 | 67.67 |
| 2 | Povidone *Ph. Eur.* | Binder | 18.00 | 4.00 |
| 3 | Sodium Lauryl Sulfate *Ph. Eur.* | Surfactant | 15.00 | 3.33 |
| 4 | Croscarmellose sodium *Ph. Eur.* | Disintegrant | 9.00 | 2.00 |
| 5 | Magnesium Stearate *Ph. Eur.* | Lubricant | 4.50 | 1.00 |

| **Stage-C (Lubrication):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium Stearate *Ph. Eur.* | Lubricant | 4.50 | 1.00 |
| **Tablet Weight (mg):** | | | 450.00 | 100.00 |

**Example E26 of NetupitantlPalonosetron 300 mg/0.5 mg tablets:** Inventive composition as tablet comprising Netupitant and Palonosetron as API and sodium lauryl sulfate as surfactant and corn starch as binder, prepared by direct compression.

| **S. No.** | **Ingredients** | **Function** | **mg/tablet** | **% by weight of tablet** |
|---|---|---|---|---|
| **Stage-A (Blending):** | | | | |
| 1 | Palonosetron Hydrochloride *In-house* | API | 0.56 | 0.12 |
| 2 | Cellulose, microcrystalline (Vivapur^{®} 102) *Ph. Eur.* | Filler | 84.94 | 18.88 |

| **Stage-B (Blending & Comilling):** | | | | |
|---|---|---|---|---|
| 1 | Netupitant *In-house* | API | 300.00 | 67.67 |
| 2 | Povidone *Ph. Eur.* | Binder | 18.00 | 4.00 |
| 3 | Sodium Lauryl Sulfate *Ph. Eur.* | Surfactant | 15.00 | 3.33 |
| 4 | Croscarmellose sodium *Ph. Eur.* | Disintegrant | 18.00 | 4.00 |
| 5 | Colloidal silicon dioxide (Aerosil^{®} 200 Pharma) *Ph. Eur.* | Glidant | 9.00 | 2.00 |

| **Stage-C (Blending):** | | | | |
|---|---|---|---|---|
| 1 | Magnesium Stearate *Ph. Eur.* | Lubricant | 4.50 | 1.00 |
| **Tablet Weight (mg):** | | | 450.00 | 100.00 |

**Example E27 of NetupitantlPalonosetron 300 mg/0.5 mg tablets:** Inventive composition as tablet comprising Netupitant and Palonosetron as API and sodium lauryl sulfate as surfactant and sodium stearyl fumarate as lubricant, prepared by wet granulation. The lubricant sodium stearyl fumarate is distributed in the intragranular and extragranular phase at 50:50 weight ratio.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 80.44 | 17.88 |
| 3 | Povidone *Ph. Eur.* | Binder | 18.00 | 4.00 |
| 4 | Sodium Lauryl Sulfate *Ph. Eur.* | Surfactant | 15.00 | 3.33 |
| 5 | Croscarmellose sodium *Ph. Eur.* | Disintegrant | 9.00 | 2.00 |
| 6 | Sodium Stearyl fumarate *Ph. Eur.* | Lubricant | 4.50 | 1.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Sodium Stearyl fumarate *Ph. Eur.* | Lubricant | 4.50 | 1.00 |
| **Tablet Weight (mg):** | | | 450.00 | 100.00 |

**Example E28 of NetupitantlPalonosetron 300 mg/0.5 mg tablets:** Inventive composition as tablet comprising Netupitant and Palonosetron as API and sodium lauryl sulfate as surfactant and sodium stearyl fumarate and magnesium stearate as lubricant, prepared by wet granulation. The lubricant sodium stearyl fumarate is placed in the intragranular phase and the lubricant magnesium stearate in the extragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Cellulose, microcrystalline (Vivapur^{®} 101) *Ph. Eur.* | Filler | 80.44 | 17.88 |
| 3 | Povidone *Ph. Eur.* | Binder | 18.00 | 4.00 |
| 4 | Sodium Lauryl Sulfate *Ph. Eur.* | Surfactant | 15.00 | 3.33 |
| 5 | Croscarmellose sodium *Ph. Eur.* | Disintegrant | 9.00 | 2.00 |
| 6 | Sodium Stearyl Fumarate *Ph. Eur.* | Lubricant | 4.50 | 1.00 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 9.00 | 2.00 |
| 2 | Colloidal silicon dioxide (Aerosil^{®} 200 Pharma) *Ph. Eur.* | Glidant | 9.00 | 2.00 |
| 3 | Magnesium stearate *Ph. Eur.* | Lubricant | 4.50 | 1.00 |
| **Tablet Weight (mg):** | | | 450.00 | 100.00 |

**Example E29 of NetupitantlPalonosetron 300 mg/0.5 mg tablets:** Inventive composition as tablet comprising Netupitant and Palonosetron as API, polysorbate 80 (Span^{®} 80) as surfactant, mannitol and microcrystalline cellulose as a filler and sodium stearyl fumarate as lubricant, prepared by wet granulation. The filler mannitol is placed in the in the intragranular phase and the filler microcrystalline cellulose in the extragranular phase. The lubricant sodium stearyl fumarate is placed in extragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.00 | 66.67 |
| 2 | Mannitol (Pearlitol^{®} 25C) *Ph. Eur.* | Filler | 30.00 | 6.67 |
| 3 | Povidone *Ph. Eur.* | Binder | 18.00 | 4.00 |
| 4 | Croscarmellose sodium *Ph. Eur.* | Disintegrant | 14.00 | 3.11 |

| **Stage-B (API solution):** | | | | |
|---|---|---|---|---|
| 1 | Palonosetron Hydrochloride *In-house* | API | 0.56 | 0.12 |
| 2 | Polysorbate 80 (Tween^{®} 80) *Ph. Eur.* | Surfactant | 15.00 | 3.33 |
| 3 | Purified water | Solvent | *q. s*. | - |

| **Stage-C (Lubrication and blending):** | | | | |
|---|---|---|---|---|
| 1 | Cellulose, microcrystalline (Vivapur^{®} 101) *Ph. Eur.* | Filler | 49.94 | 11.10 |
| 2 | Croscarmellose sodium *Ph. Eur.* | Disintegrant | 14.00 | 3.11 |
| 3 | Colloidal silicon dioxide (Aerosil^{®} 200 Pharma) *Ph. Eur.* | Glidant | 4.00 | 0.89 |
| 4 | Sodium Stearyl Fumarate *Ph. Eur.* | Lubricant | 4.50 | 1.00 |
| **Tablet Weight (mg):** | | | 450.00 | 100.00 |

**Example E30 of NetupitantlPalonosetron 300 mg/0.5 mg tablets:** Inventive composition as tablet comprising Netupitant and Palonosetron as API and sodium lauryl sulfate as surfactant, sodium stearyl fumarate and magnesium stearate as lubricant and microcrystalline cellulose and mannitol as filler, prepared by wet granulation. The lubricant sodium stearyl fumarate is placed in the intragranular phase and the lubricant magnesium stearate in the extragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 302.85 | 67.30 |
| 2 | Cellulose, microcrystalline (Vivapur^{®} 101) *Ph. Eur.* | Filler | 19.89 | 4.42 |
| 4 | Mannitol (Pearitol^{®} 25C) *Ph. Eur.* | Filler | 68.20 | 15.16 |
| 3 | Povidone *Ph. Eur.* | Binder | 13.50 | 3.00 |
| 4 | Sodium Lauryl Sulfate *Ph. Eur.* | Surfactant | 4.5 | 1.00 |
| 5 | Croscarmellose sodium *Ph. Eur.* | Disintegrant | 11.25 | 2.50 |
| 6 | Sodium Stearyl fumarate *Ph. Eur.* | Lubricant | 4.50 | 1.00 |

| **Stage-B (API solution):** | | | | |
|---|---|---|---|---|
| 1 | Palonosetron Hydrochloride *In-house* | API | 0.57 | 0.13 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 11.25 | 2.50 |
| 2 | Colloidal silicon dioxide (Aerosil^{®} 200 Pharma) *Ph. Eur.* | Glidant | 9.00 | 2.00 |
| 3 | Magnesium stearate *Ph. Eur.* | Lubricant | 4.50 | 1.00 |
| **Tablet Weight (mg):** | | | 450.00 | 100.00 |

**Example E31 of NetupitantlPalonosetron 300 mg/0.5 mg tablets:** Inventive composition as tablet comprising Netupitant and Palonosetron as API and sodium lauryl sulfate as surfactant, sodium stearyl fumarate and magnesium stearate as lubricant and microcrystalline cellulose and mannitol as filler (50:50 weight ratio), prepared by wet granulation. The lubricant sodium stearyl fumarate is placed in the intragranular phase and the lubricant magnesium stearate in the extragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.96 | 66.22 |
| 2 | Cellulose, microcrystalline (Vivapur^{®} 101) *Ph. Eur.* | Filler | 44.51 | 9.79 |
| 4 | Mannitol (Pearitol^{®} 25C) *Ph. Eur.* | Filler | 45.47 | 10.00 |
| 3 | Povidone *Ph. Eur.* | Binder | 13.50 | 2.97 |
| 4 | Sodium Lauryl Sulfate *Ph. Eur.* | Surfactant | 4.5 | 0.99 |
| 5 | Croscarmellose sodium *Ph. Eur.* | Disintegrant | 11.25 | 2.48 |
| 6 | Sodium Stearyl fumarate *Ph. Eur.* | Lubricant | 4.50 | 0.99 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 11.25 | 2.48 |
| 2 | Colloidal silicon dioxide (Aerosil^{®} 200 Pharma) *Ph. Eur.* | Glidant | 9.00 | 1.98 |
| 3 | Magnesium stearate *Ph. Eur.* | Lubricant | 4.50 | 1.98 |
| **Tablet Weight (mg):** | | | 454.50 | 100.00 |

**Example E32 of NetupitantlPalonosetron 300 mg/0.5 mg tablets:** Inventive composition as tablet comprising Netupitant and Palonosetron as API and sodium lauryl sulfate as surfactant, sodium stearyl fumarate and magnesium stearate as lubricant and microcrystalline cellulose and mannitol as filler (75:25 weight ratio), prepared by wet granulation. The lubricant sodium stearyl fumarate is placed in the intragranular phase and the lubricant magnesium stearate in the extragranular phase.

| **S. No.** | **Ingredients** | **Function** | **mg/tablet** | **% by weight of tablet** |
|---|---|---|---|---|
| **Stage-A (Granulation):** | | | | |
| 1 | Netupitant *In-house* | API | 300.96 | 66.22 |
| 2 | Cellulose, microcrystalline (Vivapur^{®} 101) *Ph. Eur.* | Filler | 67.24 | 14.79 |
| 4 | Mannitol (Pearitol^{®} 25C) *Ph. Eur.* | Filler | 22.74 | 5.00 |
| 3 | Povidone *Ph. Eur.* | Binder | 13.50 | 2.97 |
| 4 | Sodium Lauryl Sulfate *Ph. Eur.* | Surfactant | 4.5 | 0.99 |
| 5 | Croscarmellose sodium *Ph. Eur.* | Disintegrant | 11.25 | 2.48 |
| 6 | Sodium Stearyl fumarate *Ph. Eur.* | Lubricant | 4.50 | 0.99 |

| **Stage-B (API solution):** | | | | |
|---|---|---|---|---|
| 1 | Palonosetron Hydrochloride *In-house* | API | 0.56 | 0.12 |
| 2 | Purified water | Solvent | *q. s*. | - |

| **Stage-C (Lubrication and blending):** | | | | |
|---|---|---|---|---|
| 1 | Croscarmellose sodium *Ph. Eur.* | Disintegrant | 11.25 | 2.48 |
| 2 | Colloidal silicon dioxide (Aerosil^{®} 200 Pharma) *Ph. Eur.* | Glidant | 9.00 | 1.98 |
| 3 | Magnesium stearate *Ph. Eur.* | Lubricant | 4.50 | 1.98 |
| | | **Tablet Weight (mg):** | 454.50 | 100.00 |

For the compression of tablets per example E1 to E32, the used tools are 14.0 X 7.5 oval shape.

The resulting tablets have the size 14 mm x 7.5 mm, correspondingly, and a thickness of 4.5-6.8 mm.

### Part D - Reference product characterization

**Table: Physical parameters of Reference product Akynzeo^{®} 300mg/0.5 mg Capsules**

| **Product** | **Akynzeo^{®} 300mg/0.5 mg Capsules** |
|---|---|
| Generic Name | Netupitant and Palonosetron 300/0.5 mg Capsules |
| Lot No / Batch No. | 41000954 |
| Expiry Date | 12/2024 |
| Primary Pack | Alu - Alu Blister |
| Secondary Pack | Paperboard |
| Description | Hard gelatin capsule with white body and caramel cap with 'HE1' printed on the body. The capsule is filled with three tablets and one soft capsule. |
| Average weight of Capsules: | 655.90 mg |
| Lock length of capsules: | 21.2-21.7 mm |
| Diameter of capsules: | 7.6 mm |
| Average weight of soft capsule: | 123.2 mg |
| Average weight of three tablets: | 435.2 mg |
| Average weight of each individual tablet: | 145.10 mg |
| Diameter of tablet: | 6.02 mm |
| Thickness of tablet: | 4.17 mm |
| Disintegration Time of tablets: | 18 min 29 sec |
| Manufactured by and Marketing authorization holder | Helsinn Birex Pharmaceuticals Ltd, Damastown |
| | Mulhuddart |
| | Dublin 15 |
| | Ireland. |

### Part E - Dissolution study

Dissolution testing was performed as recommended for the combination of Netupitant with Palonosetron Hydrochloride in the FDA Dissolution Methods Database on the FDA webpage using the US Pharmacopeia (USP) Apparatus II (paddle) and following methods:
Netupitant: 900 ml of 0.07 M phosphate buffer pH 6.8 containing 1% SDS (SLS) with the paddle speed of 100 revolutions per minute (rpm), 75 minutes as QC sampling time.
Palonosetron Hydrochloride: 500 ml of 0.01 N HCl with paddle speed of 75 rpm, 45 minutes as QC sampling time.

**Table: Dissolution profile of Akynzeo^{®} 300/0.5 mg (B. No: 41000954) in pH 6.8 0.07 M Sodium Phosphate buffer containing 1% SDS - Netupitant**

| **Dissolution condition** | **Time (min)** | **Cumulative % Labeled Amount Dissolved - Netupitant** | | | | | | | |
|---|---|---|---|---|---|---|---|---|---|
| | | **1** | **2** | **3** | **4** | **5** | **6** | **Average** | % **RSD** |
| **Paddle, 900 ml at 100 rpm** | **5** | 3 | 4 | 3 | 5 | 4 | 8 | **5** | 41.57 |
| | **10** | 22 | 27 | 17 | 23 | 17 | 29 | **23** | 22.09 |
| | **15** | 41 | 51 | 41 | 38 | 29 | 47 | **41** | 18.46 |
| | **20** | 62 | 69 | 50 | 53 | 44 | 60 | **56** | 16.06 |
| | **30** | 82 | 90 | 87 | 75 | 65 | 82 | **80** | 11.25 |
| | **45** | 92 | 98 | 98 | 90 | 88 | 96 | **94** | 4.56 |
| | **60** | 96 | 98 | 98 | 95 | 95 | 97 | **97** | 1.43 |
| | **75** | 98 | 98 | 98 | 98 | 97 | 98 | **98** | 0.42 |
| | **90** | 99 | 98 | 99 | 99 | 98 | 98 | **99** | 0.56 |

**Table: Dissolution profile of Akynzeo^{®} 300/0.5 mg (B. No: 41000954) in 0.01 N HCl-Palonosetron**

| **Dissolution condition** | **Time (min)** | **Cumulative % Labeled Amount Dissolved-Palonosetron** | | | | | | | |
|---|---|---|---|---|---|---|---|---|---|
| | | **1** | **2** | **3** | **4** | **5** | **6** | **Average** | **% RSD** |
| **Paddle, 500 ml at 75 rpm** | **5** | 1 | 1 | 1 | 1 | 1 | 2 | **1** | 41.00 |
| | **10** | 2 | 2 | 1 | 87 | 10 | 7 | **18** | 188.33 |
| | **15** | 71 | 16 | 93 | 97 | 47 | 79 | **67** | 45.94 |
| | **20** | 93 | 77 | 98 | 94 | 96 | 84 | **89** | 8.21 |
| | **30** | 96 | 93 | 96 | 97 | 94 | 91 | **95** | 2.38 |
| | **45** | 96 | 94 | 96 | 97 | 95 | 97 | **96** | 1.22 |
| | **60** | 96 | 97 | 96 | 97 | 96 | 97 | **97** | 0.57 |
| | **Inf** | 96 | 96 | 97 | 98 | 96 | 98 | **97** | 1.01 |

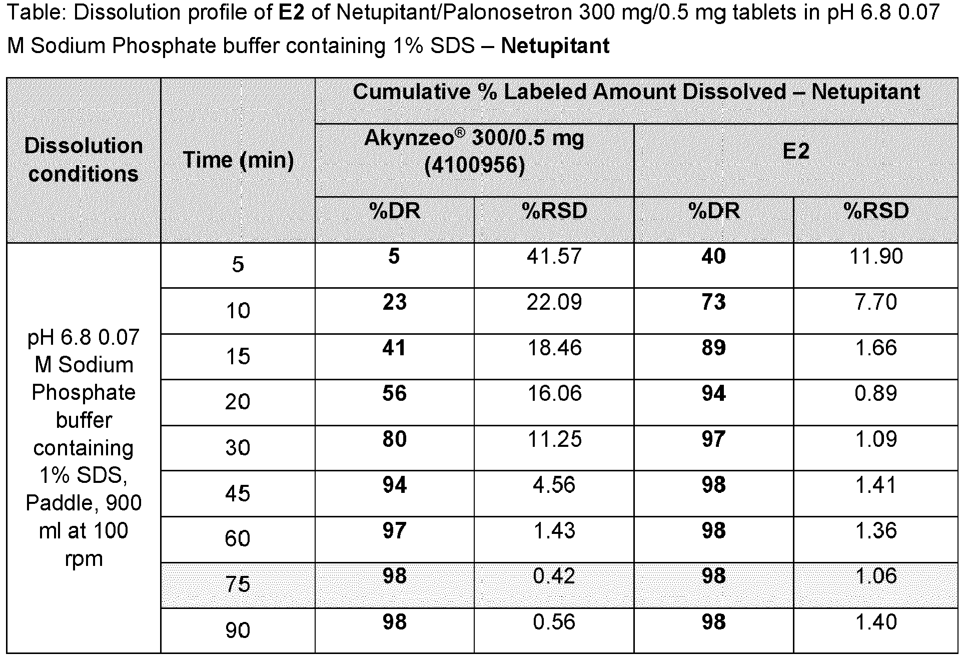

A complete release of Netupitant with more than 95% drug release is observed after 75 minutes for Akynzeo^{®} and the tablet of example E2.

According to FDA Guidance "Waiver of in vivo bioavailability and bioequivalence studies for immediate-release solid oral dosage forms based on a biopharmaceutics classification system", the dissolution results are considered highly variable if the relative standard deviation (RSD) is more than 20% at time points of 10 min or less and more than 10% at later time points.

The Netupitant dissolution results of Akynzeo^{®} at pH 6.8 is showing a high variability up to 30 minutes, whilst the inventive example E2 is demonstrating a low variability.

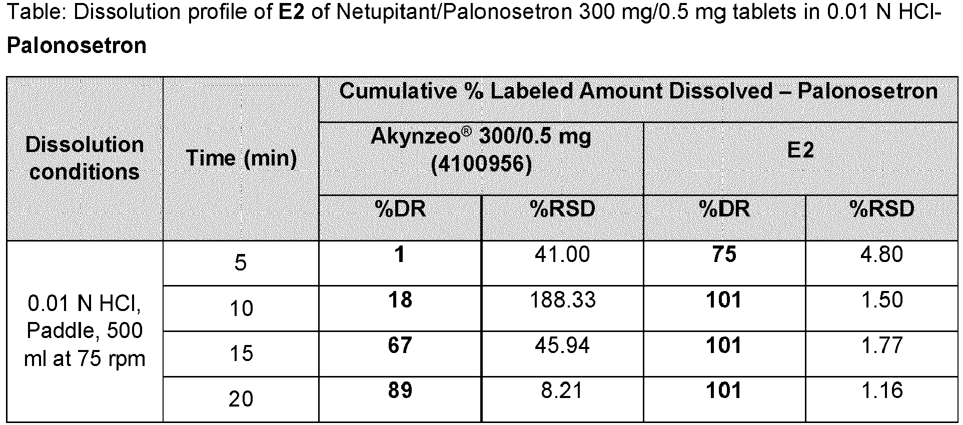

| | | | | | |
|---|---|---|---|---|---|
| | 30 | **95** | 2.38 | **102** | 1.49 |
| | 45 | **96** | 1.22 | **101** | 1.36 |
| | 60 | **97** | 0.57 | **101** | 1.61 |
| | Rec | **97** | 1.01 | **102** | 1.60 |

A complete release of Palonosetron with more than 95% drug release is observed after 45 minutes for Akynzeo^{®} and the tablet of example E2.

The Palonosetron dissolution results of Akynzeo^{®} in 0.01 N HCl media are showing a very high variability up to 15 minutes, whilst the inventive example E2 is demonstrating a low variability.

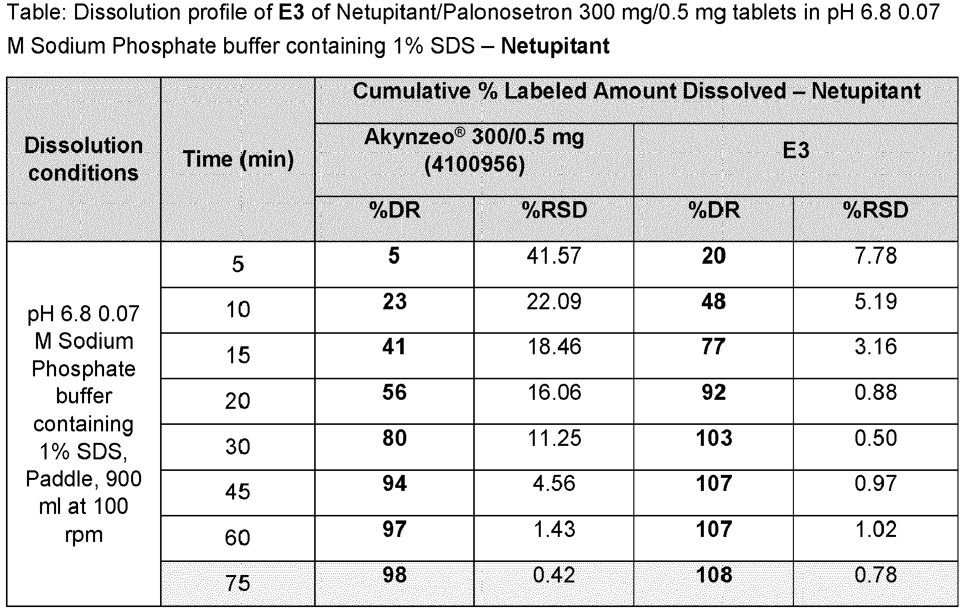

A complete release of Netupitant with more than 95% drug release is observed after 75 minutes for Akynzeo^{®} and the tablet of example E3. The dissolution of example E3 is showing a low variability.

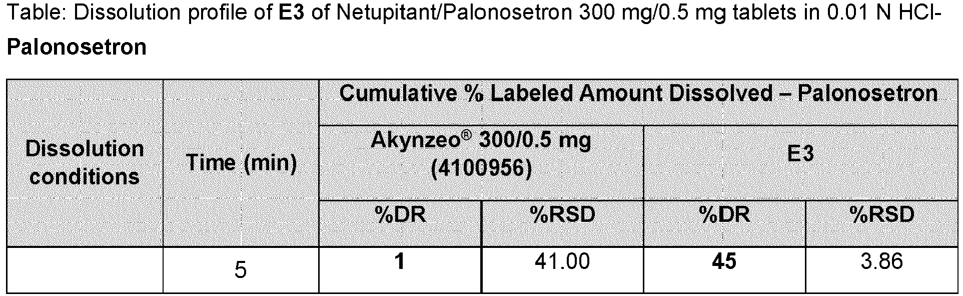

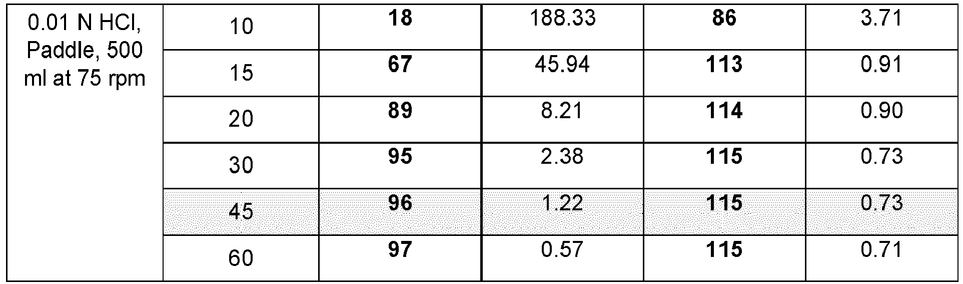

A complete release of Palonosetron with more than 95% drug release is observed after 45 minutes for Akynzeo^{®} and the tablet of example E3. The dissolution of example E3 is demonstrating a low variability.

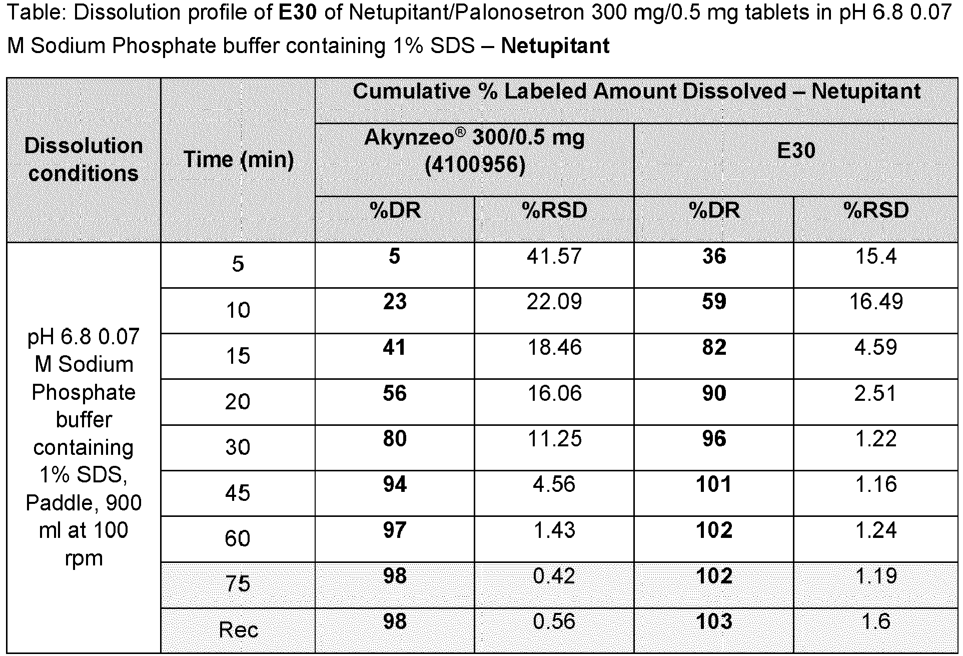

A complete release of Netupitant with more than 95% drug release is observed after 45 minutes for Akynzeo^{®} and the tablet of example E30. The dissolution of example E30 is showing a low variability.

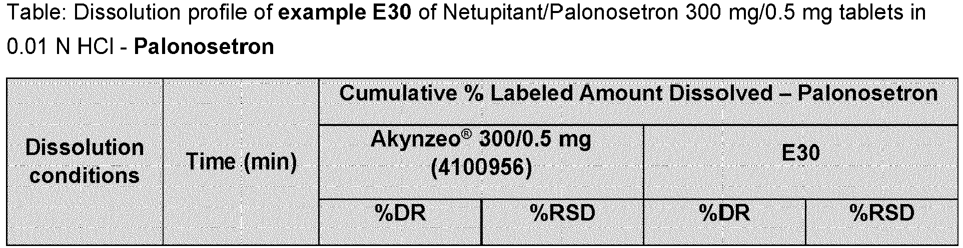

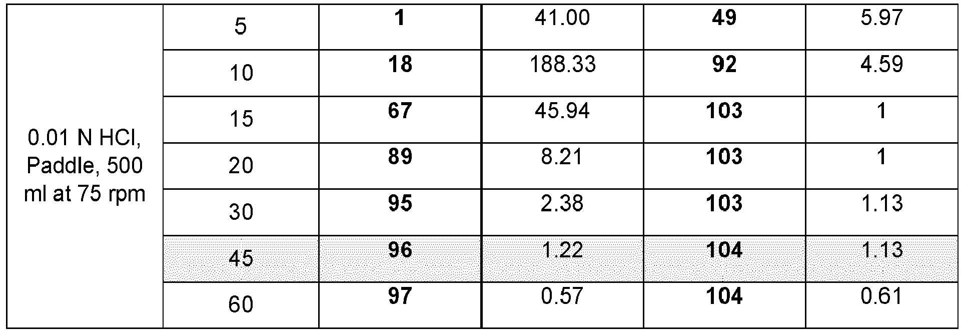

A complete release of Palonosetron with more than 95% drug release is observed after 45 minutes for Akynzeo^{®} and the tablet of example E30. The dissolution of example E30 is demonstrating a low variability.

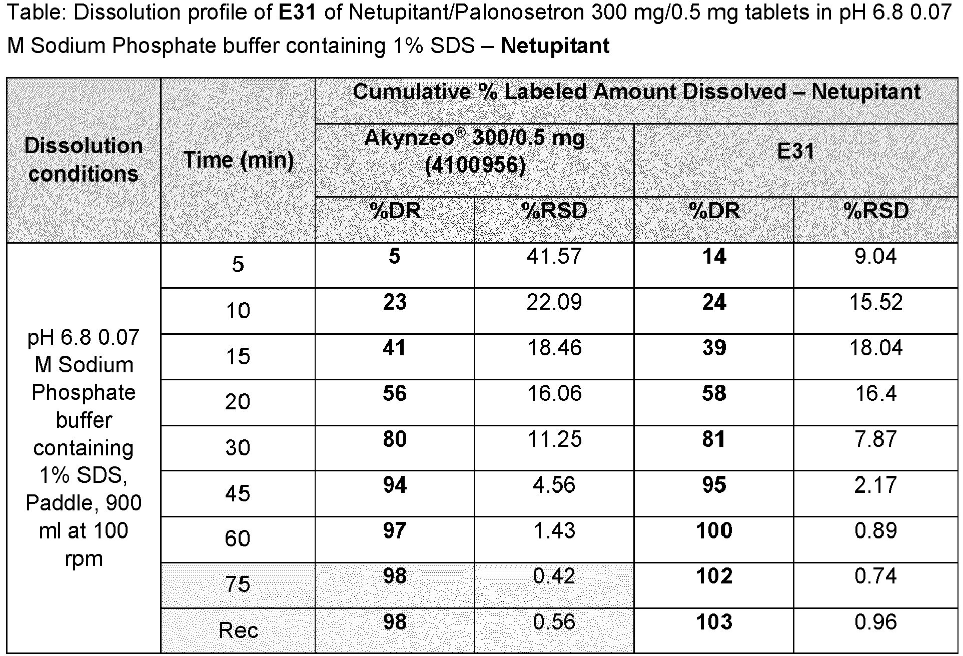

A complete release of Netupitant with more than 95% drug release is observed after 45 minutes for Akynzeo^{®} and the tablet of example E31. The dissolution of example E31 shows a lower variability in comparison to Akynzeo^{®}.

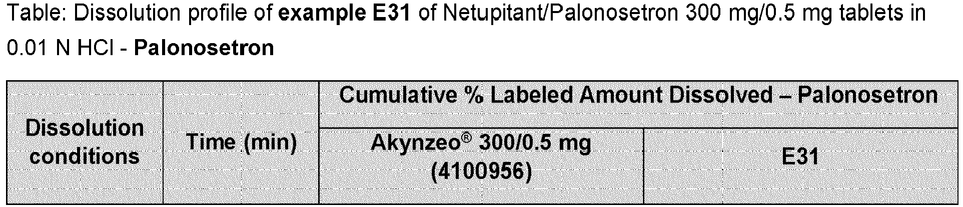

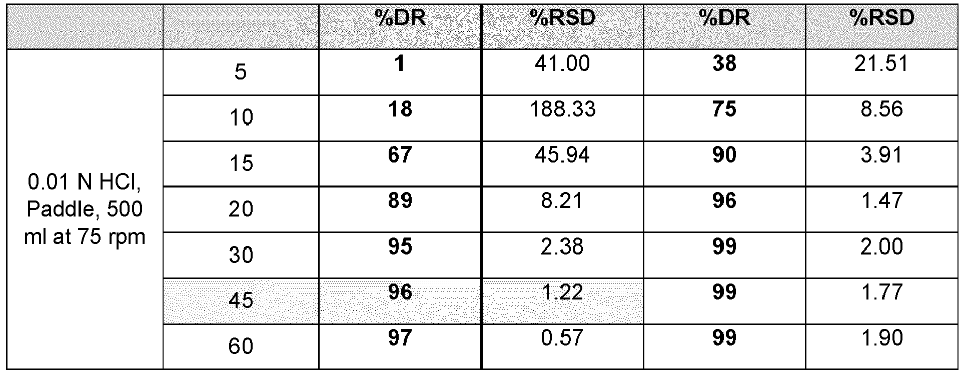

A complete release of Palonosetron with more than 95% drug release is observed after 45 minutes for Akynzeo^{®} and the tablet of example E31. The dissolution of example E31 demonstrates a low variability.

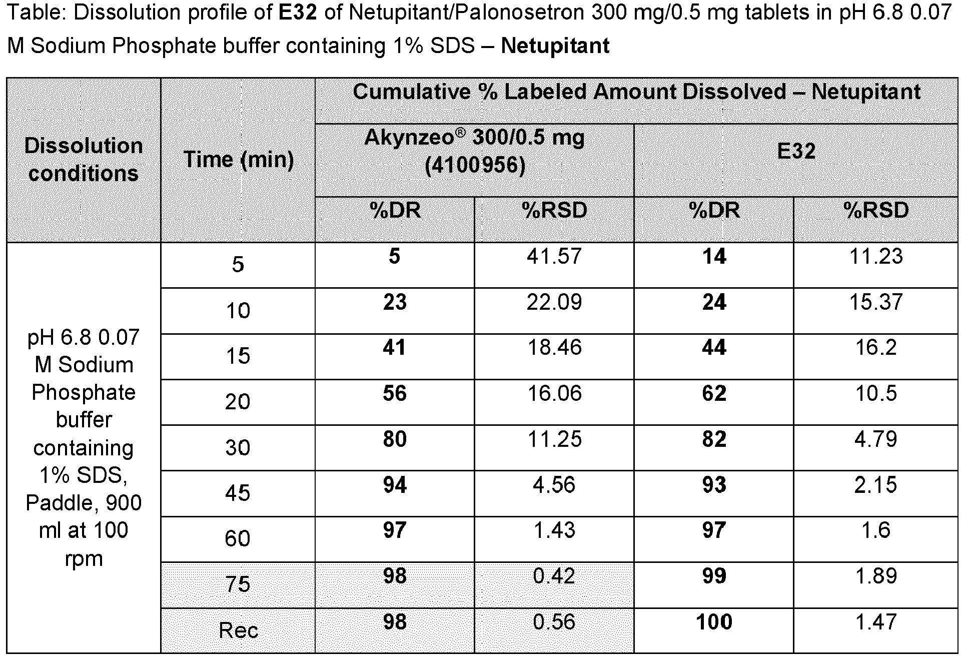

A complete release of Netupitant with more than 95% drug release is observed after 45 minutes for Akynzeo^{®} and the tablet of example E32. The dissolution of example E32 shows a lower variability in comparison to Akynzeo^{®}.

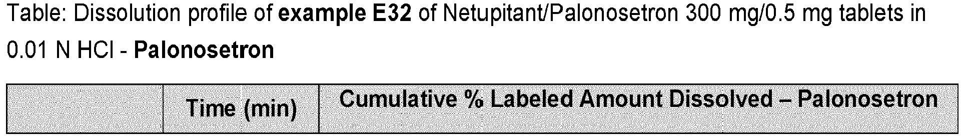

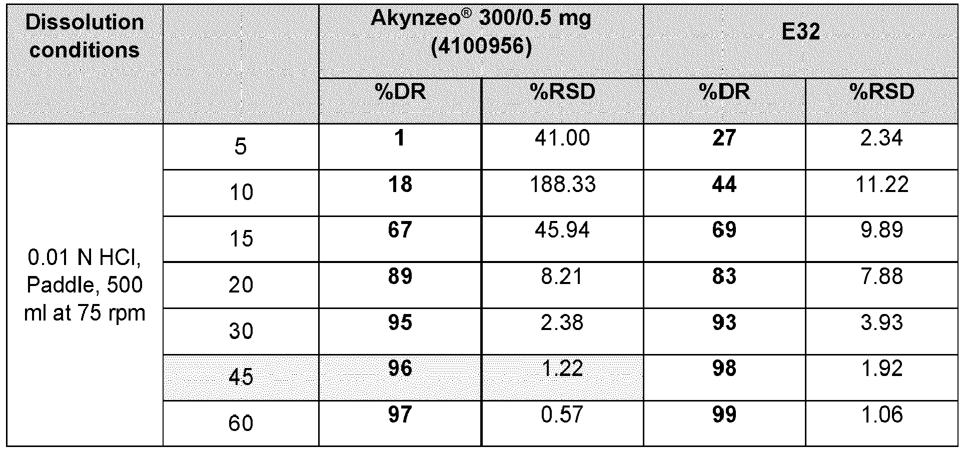

A complete release of Palonosetron with more than 95% drug release is observed after 45 minutes for Akynzeo^{®} and the tablet of example E32. The dissolution of example E32 demonstrates a low variability.

All the formulations of inventive examples E2, E3, E30, E31 and E32 showed not less than 75% dissolution of Netupitant after 75 min and not less than 75% dissolution of Palonosetron hydrochloride after 45 min in the corresponding quality control (QC) dissolution media and fulfill the regulatory standard QC test. Dissolution of the formulations of the present invention are comparable to the commercially available reference product (Akynzeo^{®}).

The Netupitant dissolution results of Akynzeo^{®} at pH 6.8 is showing a high variability up to 30 minutes, whilst the dissolution of the formulations of the present invention E2, E3, E30, E31 and E32 is demonstrating a lower variability of Netupitant drug release in comparison to Akynzeo^{®}. The Palonosetron dissolution results of Akynzeo^{®} in 0.01 N HCl media are showing a very high variability up to 15 minutes, whilst the dissolution of the formulations of the present invention E2, E3, E30, E31 and E32 is demonstrating a low variability of Palonosetron drug release.

### Part F - Content uniformity of Palonosetron

An assessment of content uniformity of tablets according to the invention was conducted. The formulations according to examples **E1** and **E2** were employed.

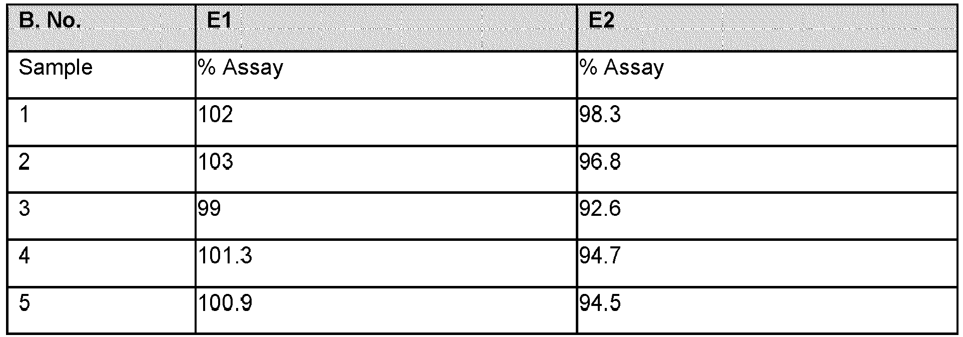

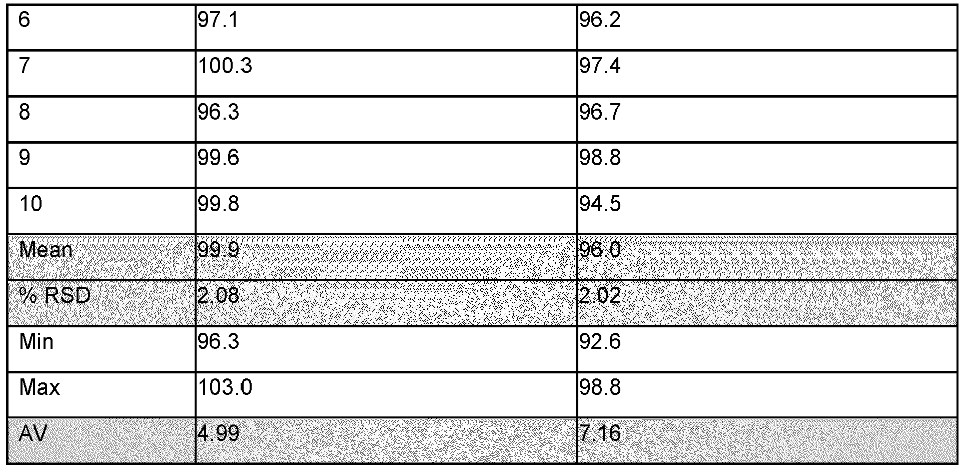

As can be observed from the data presented above, the content uniformity of Palonosetron within the formulations according to the present invention is found to be within the specification limits according to European Pharmacopeia 2.9.40. To ensure the consistency of dosage units, each unit in a batch should have an active substance content within a narrow range around the label claim. Dosage units are defined as dosage forms containing a single dose or a part of a dose of an active substance in each dosage unit. The term "Uniformity of dosage unit" is defined as the degree of uniformity in the amount of the active substance among dosage units. Therefore, the requirements of this regulation can be applied to any active substance being comprised in dosage units containing one or more active substances. The uniformity of dosage forms (by content uniformity) was assessed according to European Pharmacopeia 2.9.40.

According to the EPAR of the reference product Akynzeo^{®} co-formulations of Netupitant and Palonosetron within a single solid dosage form had poor content uniformity, in particular due to on the large difference in dose size of the active ingredients (300 vs 0.5 mg) and differences in physicochemical properties of Netupitant and Palonosetron. The formulations of the present invention thus shown an improvement over the prior art such as the reference product Akynzeo^{®} as the formulations show high content uniformity and enable the co-formulation of Netupitant and Palonosetron within a single solid pharmaceutical unit.

### Part G - Stability studies:

### Stability studies - 2 months at 40°C/75% RH

For evaluation of the impact of antioxidant on the comparative composition C1 and the inventive composition E2 are packed in Alu-Alu blisters and stored for 2 months at 40°C/75% RH (ICH accelerated conditions) according to ICH guideline Q1A(R2).

Related substances (impurities and/or degradants) of Netupitant and Palonosetron were tested at the initial stage prior to storage and after storage under conditions as shown in tables for the corresponding examples. Related substances were detected by HPLC analysis.

**Table: Related substance data of Netupitant and Palonosetron 300/0.5 mg Tablets (comparative example C1)**

| **Batch No.** | **RRT** | **Comparative example C1** | |
|---|---|---|---|
| **Condition** | | **Initial** | **40°C/75%RH-** |
| **Station** | | - | **2M** |
| 1 | 0.306 | 0.00 | 0.00 |
| 2 | 0.334 | 0.00 | 0.00 |
| 3 | 0.411 | 0.00 | 0.00 |
| 4 | 0.486 | 0.00 | 0.00 |
| 5 | 0.548 | 0.00 | 0.00 |
| 6 | 0.582 | 1.34 | 9.85 |
| 7 | 0.626 | 1.62 | 0.49 |
| 8 | 0.748 | 1.40 | 1.04 |
| 9 | Imp-E | 0.00 | 0.00 |
| 10 | Imp-A | 1.35 | 0.65 |
| 11 | 1.090 | 0.00 | 0.00 |
| 12 | 1.164 | 0.00 | 0.00 |
| 13 | 1.214 | 0.00 | 0.00 |
| 14 | 1.354 | 0.73 | 0.84 |
| 15 | **Total Imp** | **6.44** | **12.87** |

**Table: Related substance data of Netupitant and Palonosetron 300/0.5 mg Tablets (example E2)**

| **Batch No.** | **RRT** | **Example E2** | |
|---|---|---|---|
| **Condtion** | | **Initial** | **40°C/75%RH-** |
| **Station** | | **-** | **2M** |
| 1 | 0.242 | 0.00 | 0.00 |
| 2 | 0.306 | 1.18 | 0.20 |
| 3 | 0.334 | 0.15 | 0.19 |
| 4 | 0.387 | 0.00 | 0.00 |
| 5 | 0.411 | 0.00 | 0.13 |
| 6 | 0.437 | 0.00 | 0.00 |
| 7 | 0.486 | 0.09 | 0.00 |
| 8 | 0.548 | 0.00 | 0.00 |
| 9 | 0.582 | 0.00 | 0.00 |
| 10 | Imp-E | 0.00 | 0.00 |
| 11 | Imp-A | 0.06 | 0.27 |
| 12 | 1.090 | 0.10 | 0.00 |
| 13 | 1.164 | 0.00 | 0.07 |
| 14 | 1.214 | 0.13 | 0.14 |
| **15** | **Total Imp** | 1.71 | 1.00 |

As can be observed from the tables above, the novel antioxidant-free formulations of the present invention show substantially higher stability after 2 months under ICH accelerated conditions (40°C/75% RH) in Alu-Alu blister in comparison to the comparative antioxidant-containing formulation C1. Whereas the inventive antioxidant-free formulation remained stable, the comparative formulation comprising an antioxidant showed substantial higher total impurities and further the color of the tablets changed from off-white to pink upon stability studies.

### Stability studies - 6 months at 40°Cf75% RH and 6 months at 25°C/60% RH

A number of formulations are packed in Alu-Alu blisters and stored for 6 months at 40°C/75% RH (ICH accelerated conditions) and 25°C/60% RH (ICH long-term conditions) according to ICH guideline Q1A(R2).

Related substances (impurities and/or degradants) of Netupitant and Palonosetron were tested at the initial stage prior to storage and after storage under conditions as shown in tables for the corresponding examples. Related substances were detected by HPLC analysis with optimized analytical method.

**Table: Related substance data of Netupitant and Palonosetron 300/0.5 mg Tablets (example E1)**

| **S.No.** | **Impurity** | **Proposed Limit** | **E1 (40 °C/ 75% RH - 6M)** | **E1 (25 °C /60% RH - 6M)** |
|---|---|---|---|---|
| 1 | Unknown-1 | General limit: NMT 0.2 (Based on ICH: NMT 0.4) | 0.11 | 0.16 |
| 2 | Unknown-2 | | 0.19 | 0.12 |
| 3 | Unknown-3 | | 0.12 | 0.12 |
| 4 | USP RC-A | NMT 1.0 | 0.23 | 0.15 |
| % Total Impurity | | | 0.65 | 0.55 |

**Table: Related substance data of Netupitant and Palonosetron 300/0.5 mg Tablets (example E2)**

| **S.No.** | **Impurity** | **Proposed Limit** | **E2 (40 °C /75% RH - 6M)** | **E2 ( 25 °C/60% RH - 6M)** |
|---|---|---|---|---|
| 1 | Unknown-1 | General limit: NMT 0.2 (Based on ICH: NMT 0.4) | 0.06 | 0.05 |
| 2 | Unknown-2 | | 0.12 | 0.13 |
| 3 | Unknown-3 | | 0.17 | 0.17 |
| 4 | USP RC-A | NMT 1.0 | 0.37 | 0.15 |
| % Total Impurity | | | 0.72 | 0.50 |

As can be observed from the tables above, the novel formulations of the present invention show high stability after 6 months under ICH long-term conditions (25°C/60% RH) and under ICH accelerated conditions (40°C/75% RH) in Alu-Alu blister.

## Claims

1. A pharmaceutical composition comprising a fixed dose combination of (a) Netupitant or salt or hydrate thereof, and (b) Palonosetron or salt or hydrate thereof, in a single pharmaceutical unit, wherein the pharmaceutical composition does not comprise an antioxidant.

2. The pharmaceutical composition according to claim 1, in the form of a tablet.

3. The pharmaceutical composition according to any one of the preceding claims, wherein the (a) Netupitant or salt or hydrate thereof, and (b) Palonosetron or salt or hydrate thereof are present in the same granular phase.

4. The pharmaceutical composition according to claim 3, wherein the Netupitant and the Palonosetron are present in combination in an intragranular phase.

5. The pharmaceutical composition according to any one of the preceding claims, wherein the composition comprises an intragranular phase, comprising a filler, a binder and a disintegrant, and optionally a lubricant.

6. The pharmaceutical composition according to claim 5, wherein the filler is microcrystalline cellulose and/or mannitol, the binder is povidone, copovidone and/or corn starch, the disintegrant is croscarmellose sodium, sodium starch glycolate, crospovidone and/or pregelatinized starch, and the lubricant is magnesium stearate and/or sodium stearyl fumarate.

7. The pharmaceutical composition according to any one of the preceding claims, wherein the composition comprises an intragranular phase, comprising a surfactant.

8. The pharmaceutical composition according to claim 7, wherein the surfactant is sodium lauryl sulfate (SLS), poloxamer 188, sucrose laurate, polysorbate 20, polysorbate 80, sorbitan monolaurate and/or sorbitan monooleate.

9. The pharmaceutical composition according to any one of the preceding claims, wherein the composition comprises an extragranular phase, comprising a lubricant, a disintegrant and a glidant, and optionally a filler.

10. The pharmaceutical composition according to claim 9, wherein the lubricant is magnesium stearate and/or sodium stearyl fumarate, the disintegrant is croscarmellose sodium, sodium starch glycolate, crospovidone and/or pregelatinized starch, the glidant is colloidal silicon dioxide, and the filler is microcrystalline cellulose and/or mannitol.

11. The pharmaceutical composition according to any one of the preceding claims, comprising intragranular and extragranular phases, wherein a disintegrant is present in both granular phases in mass ratio of 5:1 to 1:5 of disintegrant in the intragranular to disintegrant in the extragranular phase, preferably 2:1 to 1:2, more preferably 1:1, and is preferably croscarmellose sodium, sodium starch glycolate, crospovidone and/or pregelatinized starch.

12. The pharmaceutical composition according to any one of the preceding claims, comprising
i. An intragranular combination, comprising:
a) Netupitant or a salt or hydrate thereof, present in an amount of 50-75 wt%, preferably 55-70 wt%, more preferably 58-68 wt%,
b) Palonosetron or a salt or hydrate thereof, present in an amount of 0.05-0.5 wt%, preferably 0.08-0.3 wt%, more preferably 0.1-0.2 wt%,
c) a filler, present in an amount of 2-40 wt%, preferably 5-35 wt%, more preferably 6-32 wt%,
d) a binder present in an amount of 0.1-10 wt%, preferably 0.5-9 wt%, more preferably 0.8-8 wt%
e) a disintegrant present in an amount of 0.1-5 wt%, preferably 0.5-4 wt%, more preferably 0.8-3.8 wt%,
f) optionally a surfactant in an amount of 0.1-5 wt%, preferably 0.5-4 wt%, more preferably 0.8-3.5 wt%,
g) optionally a lubricant in an amount of 0.1-5 wt%, preferably 0.5-3 wt%, more preferably 0.8-1.5 wt%,
and
ii. An extragranular component, comprising:
a) a lubricant, present in an amount of 0.1-5 wt%, preferably 0.5-3 wt%, more preferably 0.8-1.5 wt%,
b) optionally a disintegrant, present in an amount of 0.1-5 wt%, preferably 0.5-4 wt%, more preferably 0.8-3.8 wt%,
c) optionally a glidant, present in an amount of 0.1-5 wt%, preferably 0.5-3 wt%, more preferably 0.8-2.5 wt%, and
d) optionally a filler, in an amount of 1-30 wt%, preferably 5-20 wt%, more preferably 10-15 wt%
wherein wt% values are based on the total weight of all components of the tablet.

13. The pharmaceutical composition according to any one of the preceding claims, wherein said composition is prepared by
i. wet granulating a dry mixture comprising Netupitant or a salt or hydrate thereof, filler, a binder, a disintegrant, and optionally a surfactant and/or a lubricant, with a solution comprising Palonosetron or a salt or hydrate thereof, and optionally a surfactant and/or a binder, to produce granules,
ii. blending the granules with a lubricant, a disintegrant and a glidant, and optionally a filler, and
iii. compressing of the lubricated granules to produce a tablet.

14. The pharmaceutical composition according to any one of the preceding claims, wherein the tablet is not coated.

15. The pharmaceutical composition according to any one of the preceding claims, wherein the tablet shows at least 75% dissolution of Netupitant in the dissolution test using the USP Apparatus II in 900 ml of 0.07 M phosphate buffer pH 6.8 containing 1% SDS with the paddle speed of 100 rpm after 75 minutes and/or at least 75% dissolution of Palonosetron using the USP Apparatus II in 500 ml of 0.01 N HCl with the paddle speed of 75 rpm after 45 minutes.

16. The pharmaceutical composition according to any one of the preceding claims, wherein the tablet shows content uniformity (CU) between 85% and 115% (based on assay by HPLC analysis) and the acceptance value (AV) less than 15 for Palonosetron according to the content uniformity test of the European Pharmacopeia (Ph. Eur.) 2.9.40.

17. The pharmaceutical composition according to any one of the preceding claims for use in the treatment and/or prevention of nausea and/or vomiting, preferably in the treatment and/or prevention of acute or delayed nausea and vomiting associated with chemotherapy, such as highly or moderately emetogenic cancer chemotherapy, for example cisplatin cancer chemotherapy.

18. A method of preparing a pharmaceutical composition in the form of a tablet, the method comprising:
i. wet granulating a dry mixture comprising Netupitant or a salt or hydrate thereof, filler, a binder, a disintegrant, and optionally a surfactant and/or a lubricant, with a solution comprising Palonosetron or a salt or hydrate thereof, and optionally a surfactant and/or a binder, to produce granules,
ii. blending the granules with a lubricant, a disintegrant and a glidant, and optionally a filler, and
iii. compressing of the lubricated granules to produce a tablet.
